# FASCICULE DE BREVET EUROPEEN

(11) **EP 3 935 468 B1**
(45) Date de publication et mention de la délivrance du brevet: **15.03.2023**
(21) Numéro de dépôt: 20704339.9
(22) Date de dépôt: 18.02.2020
(51) Int. Cl.: G05D 11/13, G01N 33/00

(54) **GÉNÉRATEUR MICROFLUIDIQUE DE MÉLANGE GAZEUX**
MIKROFLUIDISCHER GENERATOR ZUR ERZEUGUNG EINES GASGEMISCHES
MICROFLUIDIC GENERATOR FOR GENERATING A GAS MIXTURE

(30) Priorité: 06.03.2019 FR 1902269
(43) Date de publication de la demande: 12.01.2022
(73) Titulaire: Centre national de la recherche scientifique, 75016 Paris (FR); In' Air Solutions, 67000 Strasbourg (FR); Université de Strasbourg, 67000 Strasbourg (FR)
(72) Inventeur: LECALVE, Stéphane, 67117 ITTENHEIM (FR); NOEL, Florian, 88380 ARCHES (FR); SERRA, Christophe, 67460 SOUFFELWEYERSHEIM (FR)
(74) Mandataire: Atout PI Laplace
(86) Numéro de dépôt international: PCT/EP2020/054244
(87) Numéro de publication internationale: WO 2020/178022

(56) Documents cités:
- WO-A1-95/34851
- US-A- 5 239 856
- US-A1- 2004 130 965

## Description

La présente invention concerne un procédé de génération d'un mélange gazeux et un générateur de mélange gazeux adapté pour mettre en oeuvre ce procédé.

Certains mélanges gazeux, en particulier des mélanges comprenant des composés volatils organiques, de l'ammoniac (NH₃) ou du dioxyde de carbone (CO₂), sont utilisés pour la calibration d'instruments d'analyse de la qualité de l'air.

Ces instruments d'analyse sont utilisés pour mesurer la quantité de polluant dans l'air. Il faut donc leur fournir un mélange gazeux comprenant une concentration connue de polluant pour leur calibration.

Généralement, ces mélanges sont générés à partir d'un gaz étalon dilué dans un flux de gaz supplémentaire, ou par le passage d'un gaz porteur du polluant à travers une chambre de perméation régulée en température, ou à partir d'un tube à perméation quand on souhaite réaliser un mélange dilué à partir d'un solution aqueuse, le gaz porteur passant dans le tube à perméation, ou encore à partir d'une solution liquide injectée dans un évaporateur à 200 °C dont la vapeur est ensuite emportée par un flux de gaz porteur.
Le brevet US5239856 décrit la génération de mélanges de gaz étalons et, plus particulièrement, un procédé et un appareil de génération de mélanges de gaz étalons pour tester, étalonner des instruments d'analyse hautement sensibles tels qu'un spectromètre de masse pour de l'ionisation chimique à pression atmosphérique.
La demande de brevet US 2004/0130965 A1 décrit des méthodes de dilution d'un produit chimique utilisé en relation avec la fabrication de dispositifs semi-conducteurs et décrit plus particulièrement un volume fixe connecté entre une alimentation en produits chimiques et un réservoir de stockage.

Les inconvénients de ces méthodes de génération ou des appareils permettant la génération de ces mélanges est que leur poids est élevé et/ou qu'ils nécessitent des débits élevés en gaz porteur pour obtenir des concentrations faibles en polluant dans le mélange et/ou qu'ils génèrent des concentrations élevées en polluant dans le mélange. La nécessité d'avoir des débits élevés ou des concentrations élevées engendre des pertes importantes en gaz porteur et/ou polluant et de fait, réduit drastiquement l'autonomie ou alors nécessite des quantités de gaz porteur ou de polluant importantes.

Par ailleurs, le poids et donc la portabilité des appareils déjà existants sont liés au poids du matériel et/ou au poids des bouteilles de gaz porteur (comme l'air) volumineuses qui sont indispensables pour générer les débits de gaz porteur nécessaires (entre 1000 et 5000 mL/min). Certains appareils sont néanmoins légers, mais ils nécessitent tout de même des débits importants de gaz porteur.

L'invention vise à remédier aux inconvénients précités de l'art antérieur, plus particulièrement elle vise à proposer un procédé de génération de mélange gazeux et un générateur de mélange gazeux pouvant mettre en oeuvre le procédé permettant d'obtenir de faibles débits, ayant un poids faible et une faible consommation en gaz porteur.

Un objet de l'invention est donc un procédé de génération d'un mélange gazeux au moyen d'un appareil comprenant au moins deux entrées, dont la première est une entrée pour un gaz porteur et la seconde est une entrée pour un polluant et au moins une sortie de gaz, un système d'électrovannes, un circuit microfluidique et une cellule de mélange, le circuit microfluidique comprenant un sous-circuit pouvant être isolé ou mis en relation avec la cellule de mélange grâce au système d'électrovannes, caractérisé en ce qu'il comprend les étapes suivantes :
a) Nettoyage du circuit microfluidique par de l'air pur reçu sur la première entrée ;
b) Formation d'un premier train d'air avec un gaz reçu sur la première entrée de l'appareil, envoi de ce premier train d'air vers la cellule de mélange et ajout d'un polluant dans le sous-circuit isolé de la cellule de mélange à partir d'au moins un polluant reçu sur la seconde entrée de l'appareil ;
c) Ouverture du sous-circuit par le système d'électrovannes, de manière à ce que le sous-circuit soit relié à la première entrée de l'appareil alimentée en gaz et à l'entrée de la cellule de mélange, l'ouverture du sous-circuit provoquant l'envoi d'un second train d'air vers la cellule de mélange ;
   les étapes b) et c) étant répétées jusqu'à obtenir la quantité de mélange gazeux souhaitée en sortie de la cellule de mélange.

Selon des modes de réalisation de l'invention :
- le polluant ajouté dans le sous-circuit isolé durant l'étape b) est gazeux et l'étape b) comprend également l'homogénéisation de la pression en polluant gazeux dans le sous-circuit ; et
- le polluant ajouté durant l'étape b) est liquide et l'ajout de ce polluant est réalisé par le dépôt d'une goutte (G) ou le dépôt séquentiel d'au moins deux gouttes du polluant dans le sous-circuit et durant l'étape c), l'ouverture du sous-circuit provoque l'évaporation des gouttes dans le gaz venant de la première entrée de l'appareil.

Un second objet de l'invention est un générateur de mélange gazeux pour la mise en oeuvre du procédé selon l'invention comprenant :
- un régulateur de débit massique placé à une première entrée de gaz du générateur ;
- une première électrovanne 3 voies dont la première voie est placée à une seconde entrée de gaz du générateur ;
- un régulateur de pression placé entre la première voie de l'électrovanne 3 voies et la seconde entrée de gaz du générateur ;
- une cellule de mélange présentant une entrée et une sortie et comprenant au moins une zone tampon comprenant une entrée et une sortie, l'entrée de la zone tampon étant reliée à l'entrée de la cellule et la sortie de la zone tampon étant reliée à la sortie de la cellule et la sortie de la cellule formant une sortie d'un mélange gazeux du générateur ; et
- une électrovanne 6 voies dont une première voie est reliée à une sortie du régulateur de débit massique, une seconde voie est reliée à l'entrée de la cellule de mélange, une troisième voie est reliée à une seconde voie de la première électrovanne 3 voies, une quatrième et une cinquième voies sont reliées ensemble et une sixième voie est reliée à la troisième voie de l'électrovanne 3 voies.

Selon des modes de réalisation de l'invention, ce générateur de mélange gazeux peut également comprendre :
- une cellule d'évaporation présentant une entrée de gaz, une entrée de liquide et une sortie de gaz ; une seconde et une troisième électrovannes 3 voies, une première voie de la seconde électrovanne 3 voies étant reliée au régulateur de débit massique, une seconde voie de la seconde électrovanne 3 voies étant reliée à la première voie de l'électrovanne 6 voies, une troisième voie de la seconde électrovanne 3 voies étant reliée à l'entrée de gaz de la cellule d'évaporation, une première voie de la troisième électrovanne étant reliée à la seconde voie de l'électrovanne 6 voies, une seconde voie de la troisième électrovanne étant reliée à l'entrée de la cellule de mélange et une troisième voie de la troisième électrovanne 3 voies étant reliée à la sortie de la cellule d'évaporation ; et un dispositif de génération de gouttes placé à l'entrée de liquide de la cellule de mélange et configurée de manière à former une entrée du générateur pour un liquide ;
- un dispositif de génération de gouttes relié à la quatrième voie de l'électrovanne 6 voies et à la cinquième voie de l'électrovanne 6 voies, et configurée de manière à former une entrée du générateur pour un liquide ;
- un dispositif de génération de gouttes relié à la troisième voie de l'électrovanne 6 voies et à la seconde voie de la première électrovanne 3 voies, et configurée de manière à former une entrée du générateur pour un liquide ; et
- un dispositif de génération de gouttes choisi parmi une seringue, une tête d'impression ou une puce microfluidique.

Un troisième objet de l'invention est un générateur de mélange gazeux pour la mise en oeuvre du procédé selon l'invention comprenant :
- un régulateur de débit massique placé à une première entrée de gaz du générateur ;
- une première électrovanne 3 voies dont la première voie est placée à une seconde entrée de gaz du générateur ;
- un régulateur de pression placé entre la première voie de l'électrovanne 3 voies et la seconde entrée de gaz du générateur ;
- une cellule de mélange présentant une entrée et une sortie et comprenant au moins une zone tampon comprenant une entrée et une sortie, l'entrée de la zone tampon étant reliée à l'entrée de la cellule et la sortie de la zone tampon étant reliée à la sortie de la cellule et la sortie de la cellule formant une sortie d'un mélange gazeux du générateur ; et
- une électrovanne 4 voies dont une première voie est reliée à une sortie du régulateur de débit massique, une seconde voie est reliée à une entrée de la cellule de mélange, une troisième voie est reliée à une seconde voie de la première électrovanne 3 voies et une quatrième voie est reliée à une troisième voie de la première électrovanne 3 voies.

Selon des modes de réalisation, ce générateur de mélange gazeux peut également comprendre :
- une cellule d'évaporation présentant une entrée de gaz, une entrée de liquide et une sortie de gaz ; une seconde et une troisième électrovannes 3 voies, une première voie de la seconde électrovanne 3 voies étant reliée au régulateur de débit massique, une seconde voie de la seconde électrovanne 3 voies étant reliée à la première voie de l'électrovanne 4 voies, une troisième voie de la seconde électrovanne 3 voies étant reliée à l'entrée de gaz de la cellule d'évaporation, une première voie de la troisième électrovanne 3 voies étant reliée à la seconde voie de l'électrovanne 4 voies, une seconde voie de la troisième électrovanne 3 voies étant reliée à l'entrée de la cellule de mélange et une troisième voie de la troisième électrovanne 3 voies étant reliée à la sortie de la cellule d'évaporation ; et un dispositif de génération de gouttes placé à l'entrée de liquide de la cellule de mélange et configurée de manière à former une entrée du générateur pour un liquide ;
- un dispositif de génération de gouttes relié à la troisième voie de l'électrovanne 4 voies et à la quatrième voie de l'électrovanne 4 voies et configurée de manière à former une entrée du générateur pour un liquide ; et
- un dispositif de génération de gouttes choisi parmi une seringue, une tête d'impression ou une puce microfluidique.

Un quatrième objet de l'invention est un générateur de mélange gazeux pour la mise en oeuvre du procédé selon l'invention comprenant :
- un régulateur de débit massique (RDM) relié à une première entrée (IN1) du générateur ;
- une cellule de mélange (C_MEL) présentant une entrée (IN_MEL) et une sortie (OUT_MEL) et comprenant au moins une zone tampon (Z1, Z2, Z3, Z4) comprenant une entrée et une sortie, l'entrée de la zone tampon étant reliée à l'entrée de la cellule et la sortie de la zone tampon étant reliée à la sortie de la cellule et la sortie de la cellule formant une sortie d'un mélange gazeux du générateur ;
- cinq électrovannes 2 voies (E21, E22, E23, E24, E25) ;
- un régulateur de pression (RDP) ; et
- un raccord en té (TE),
   la sortie du régulateur de débit massique étant reliée à une première voie (11, 12) de la première (E21) et de la seconde (E22) électrovanne, une seconde voie (21) de la première électrovanne étant reliée à une première voie (25) de la cinquième électrovanne (E25), une seconde voie (22) de la seconde électrovanne (E22) étant reliée à une première entrée (T2) du raccord en té, une première voie (13) de la troisième électrovanne (E23) étant reliée à une seconde entrée du générateur (IN2), une seconde voie (23) de la troisième électrovanne (E23) étant reliée à une deuxième entrée (T1) du raccord en té, une première voie (14) de la quatrième et une seconde voie (15) de la cinquième électrovanne (E25) étant reliées à l'entrée de la cellule de mélange (C_MEL), une seconde voie (24) de la quatrième électrovanne (E24) étant reliée à la troisième entrée (T3) du raccord en té, le régulateur de pression (RDP) étant placé entre la première voie (13) de la troisième électrovanne (E23) et la seconde entrée du générateur (IN2) et le capteur de pression (P) étant placé entre la seconde voie (24) de la quatrième électrovanne (E24) et la troisième entrée (T3) du raccord en té.

Un cinquième objet de l'invention est un générateur de mélange gazeux pour la mise en oeuvre du procédé selon l'invention comprenant :
- un régulateur de débit massique (RDM) placé à une première entrée de gaz (IN1) du générateur ;
- une première électrovanne 3 voies (E34) dont la première voie (134) est placée à une seconde entrée de gaz (IN2) du générateur ;
- un régulateur de pression (RDP) placé entre la première voie (134) de la première électrovanne 3 voies et la seconde entrée de gaz du générateur ;
- une seconde électrovanne 3 voies (E33) dont la première voie (133) est placée à une sortie du régulateur de débit massique et la seconde voie (233) est reliée à la troisième voie (334) de la première électrovanne ;
- une troisième électrovanne 3 voies (E35) dont la troisième voie (335) est reliée à la troisième voie (333) de la seconde électrovanne, la première voie (135) est reliée à la seconde voie (234) de la première électrovanne ; et
- une cellule de mélange (C_MEL) présentant une entrée (IN_MEL) et une sortie (OUT_MEL) et comprenant au moins une zone tampon (Z1, Z2, Z3, Z4) comprenant une entrée et une sortie, l'entrée de la zone tampon étant reliée à l'entrée de la cellule, la sortie de la zone tampon étant reliée à la sortie de la cellule, la sortie de la cellule formant une sortie d'un mélange gazeux du générateur, et l'entrée de la cellule étant reliée à la seconde voie (235) de la troisième électrovanne.

Les différents générateur de mélange gazeux selon l'invention peuvent comprendre :
- un capteur de pression (P) configuré de manière à mesurer la pression en polluant issu de la seconde entrée (IN2) en gaz du générateur et un système informatique (PC) pour piloter le générateur, le système informatique étant configuré pour recevoir en entrée des mesures du capteur de pression, des valeurs de débit du régulateur de débit massique (RDM), des valeurs de pression du régulateur de pression (RDP), pour contrôler le régulateur de débit massique (RDM) et le régulateur de pression (RDP) et pour contrôler les ouvertures des voies des électrovannes (E21, E22, E23, E24, E25, E3, E31, E32, E33, E35, E36, E4, E6) du générateur.

Dans ce cas :
- le système informatique peut également être configuré pour contrôler le dispositif de génération de gouttes ;
- la cellule de mélange peut comprendre au moins deux zones tampon (Z1, Z2, Z3, Z4), chacune des zones tampon étant reliées à l'entrée de la cellule de mélange (IN_MEL) et à la sortie de la cellule de mélange (OUT_MEL) ; et
- la cellule de mélange peut être multi-étages, chaque étage (Et1, Et2, Et3, Et4) de la cellule comprenant une entrée (IN_Et1, IN_Et2, IN_Et3, IN_Et4), une sortie (OUT_Et1, OUT_Et2, OUT_Et3, OUT_Et4) et au moins une zone tampon, l'entrée d'un étage étant reliée à la sortie d'un autre étage, l'entrée du premier étage de la cellule étant reliée à l'entrée de la cellule et la sortie du dernier étage de la cellule étant reliée à la sortie de la cellule (OUT_MEL).

D'autres caractéristiques, détails et avantages de l'invention ressortiront à la lecture de la description faite en référence aux figures annexées données à titre d'exemple et qui représentent, respectivement :
[Fig.1], un schéma des étapes du procédé selon l'invention ;
[Fig.2a] à [Fig. 2d], la mise en oeuvre des étapes du procédé de l'invention avec un premier type de générateur de mélange gazeux pour un polluant gazeux ;
[Fig. 3a] à [Fig. 3d], la mise en oeuvre des étapes du procédé de l'invention avec un second type de générateur de mélange gazeux pour un polluant gazeux ;
[Fig. 4], un troisième type de générateur de mélange gazeux pour la mise en oeuvre du procédé selon l'invention pour un polluant gazeux ;
[Fig. 5a] à [Fig. 5e], différents types de générateur de mélange gazeux pour la mise en oeuvre du procédé selon l'invention pour un polluant liquide ;
[Fig. 6a] à [Fig. 6c], un quatrième type de générateur de mélange gazeux pour la mise en oeuvre du procédé selon l'invention pour un polluant gazeux ; et
[Fig. 7a] à [Fig. 7e], des exemples de cellule de mélange utilisés par le procédé et/ou par les générateurs de mélange gazeux.

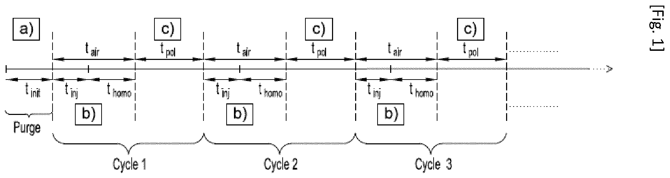

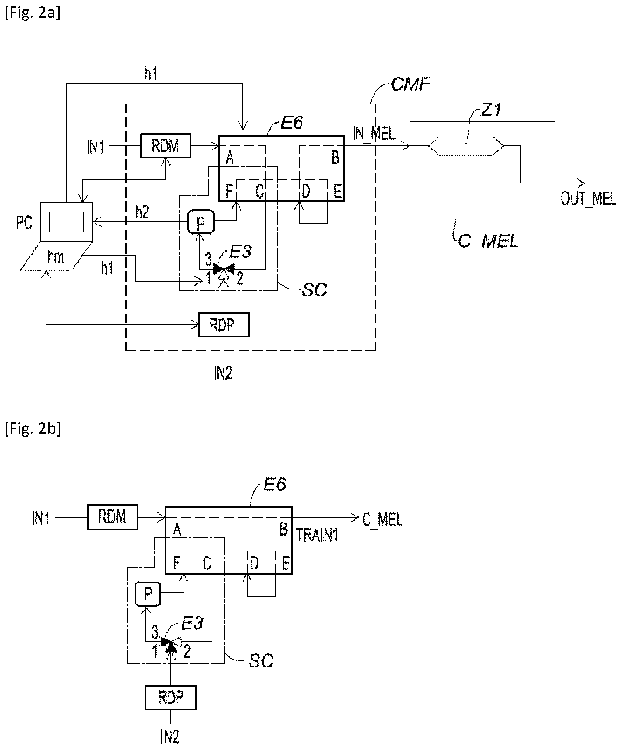

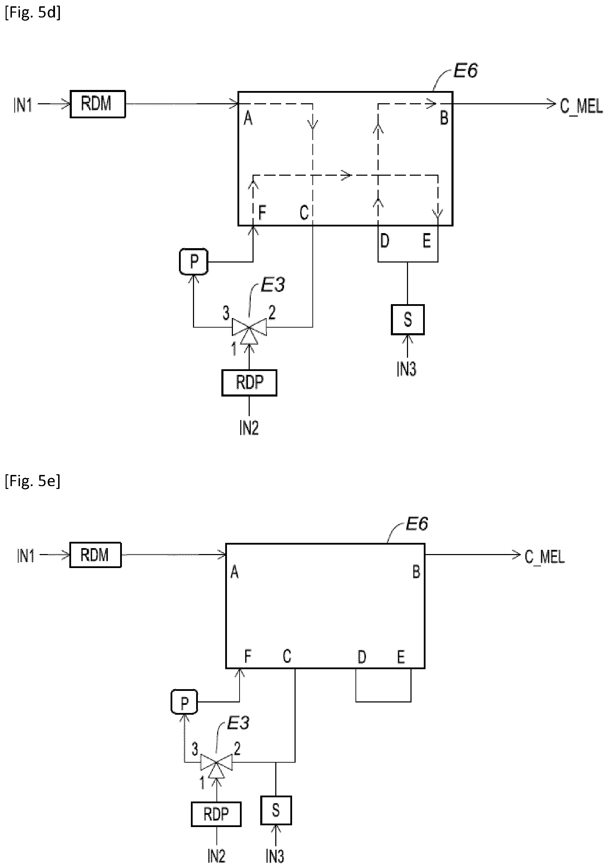

[Fig. 1] représente un schéma des étapes du procédé de génération d'un mélange gazeux selon l'invention. Le procédé comporte trois étapes numérotées de a) à c). Les étapes b) et c) peuvent être répétées plusieurs fois jusqu'à obtenir la quantité de mélange gazeux souhaitée. La succession des étapes b) et c) constitue un cycle, et la [Fig. 1] présente trois cycles (Cycle 1, Cycle 2, Cycle 3). Le procédé est mis en oeuvre au moyen d'un appareil comprenant au moins deux entrées dont la première est une entrée de gaz et la seconde est une entrée d'un polluant, au moins une sortie de gaz, un système d'électrovannes, un circuit microfluidique, comprenant lui-même un sous-circuit et une cellule de mélange. Le sous-circuit peut être isolé ou mis en relation avec la cellule de mélange grâce au système d'électrovannes. Des exemples de ce type d'appareil, dit générateur de mélange gazeux, sont décrits en référence aux [Fig. 2a] à [Fig. 5e].

La première étape du procédé, étape a), consiste à nettoyer le circuit microfluidique de l'appareil avec de l'air pur reçu sur la première entrée de gaz de l'appareil. On peut également appeler cette étape Purge. On note sa durée tᵢₙᵢₜ.

Durant l'étape b), on forme un premier train d'air avec un gaz reçu sur la première entrée de l'appareil et ce premier train d'air est envoyé vers la cellule de mélange. Préférentiellement, ce premier train d'air comprend de l'air pur. Pendant la formation et l'envoi de ce premier train d'air, on ajoute un polluant dans le sous-circuit fermé de l'appareil à partir d'un polluant reçu sur la seconde entrée de l'appareil. Ce polluant peut être gazeux, par exemple un composé volatil organique, ou liquide. La durée de cette étape est notée tₐᵢᵣ. Durant l'étape suivante, étape c), on ouvre le sous-circuit, de manière à ce qu'il soit relié à la première entrée de gaz de l'appareil et à l'entrée de la cellule de mélange. Cela a pour effet de stopper l'envoi du premier train d'air vers la cellule de mélange et de provoquer l'envoi d'un second train d'air comprenant le polluant vers la cellule de mélange. La durée de l'étape c) est notée tₚₒₗ. La durée totale d'un cycle vaut donc t_{cycle} = tₐᵢᵣ + tₚₒₗ.

Selon un mode de réalisation, le polluant ajouté dans l'étape b) est gazeux. Dans ce cas, pendant que le premier train d'air est formé et envoyé vers la cellule de mélange, on ajoute le polluant gazeux dans le sous-circuit fermé pendant une durée notée tᵢₙⱼ, puis on stoppe l'ajout pour que la pression en polluant s'homogénéise dans le sous-circuit pendant une durée notée tₕₒₘₒ. La durée de l'étape b) vaut donc tₐᵢᵣ = tᵢₙⱼ + tₕₒₘₒ.

Selon un autre mode de réalisation, le polluant ajouté dans l'étape b) est liquide. Dans ce cas, l'ajout de ce polluant liquide est réalisé par le dépôt d'une goutte ou le dépôt séquentiel d'au moins deux gouttes du polluant liquide dans le sous-circuit. Durant l'étape c), l'ouverture du sous-circuit provoque l'évaporation des gouttes dans le gaz reçu à la première entrée de l'appareil. Cela permet de générer un mélange gazeux à partir d'un liquide, par exemple de générer un mélange gazeux comprenant du formaldéhyde. Plus on génère des gouttes petites, plus on pourra contrôler précisément la quantité de polluant injectée. Les gouttes générées sont plus petites que la section du sous-circuit dans lequel elles sont injectées.

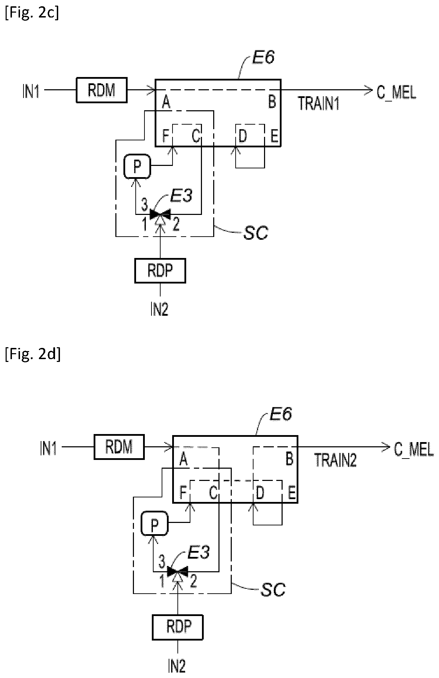

[Fig. 2a] à [Fig. 2d] présentent un générateur de mélange gazeux adapté pour la mise en oeuvre du procédé selon l'invention avec un polluant gazeux, et plus particulièrement le fonctionnement du générateur selon les étapes du procédé.

[Fig. 2a] présente le fonctionnement d'un générateur lors de l'étape de Purge (étape a)). Le générateur comprend un régulateur de débit massique RDM placé à une première entrée de gaz du générateur IN1. Ce régulateur RDM est relié à une première voie A d'une électrovanne à 6 voies E6. La deuxième entrée IN2 de gaz du générateur est reliée à une première voie 1 d'une électrovanne à 3 voies E3. La troisième voie C de l'électrovanne à 6 voies E6 est reliée à la seconde voie 2 de l'électrovanne à 3 voies E3 et la troisième voie 3 de l'électrovanne à 3 voies E3 est reliée à la sixième voie F de l'électrovanne à 6 voies E6. Un capteur de pression P est placé entre la troisième voie 3 de l'électrovanne E3 et la sixième voie F de l'électrovanne E6. Il permet de mesurer la pression du sous-circuit SC quand celui-ci est fermé dans l'étape b) du procédé, le sous-circuit SC étant formé par le chemin parcouru par le gaz, arrivant sur la seconde entrée IN2, dans l'électrovanne à 3 voies E3 et dans les sixième F et troisième C voies de l'électrovanne à 6 voies. Un régulateur de pression RDP est placé entre l'entrée IN2 et la première voie 1 de l'électrovanne E3 pour contrôler la quantité de gaz injectée dans le sous-circuit SC. L'ensemble électrovanne à 6 voies E6, électrovanne à 3 voies E3, capteur de pression P, régulateur de pression RDP et régulateur de débit massique RDM forme le circuit microfluidique CMF du générateur.

Le régulateur de débit massique RDM permet d'adapter le débit du gaz arrivant à la première entrée IN1 du générateur ce qui permet d'envoyer plus ou moins de gaz dans le générateur selon la quantité et la concentration désirée en gaz dans le mélange gazeux en sortie du générateur.

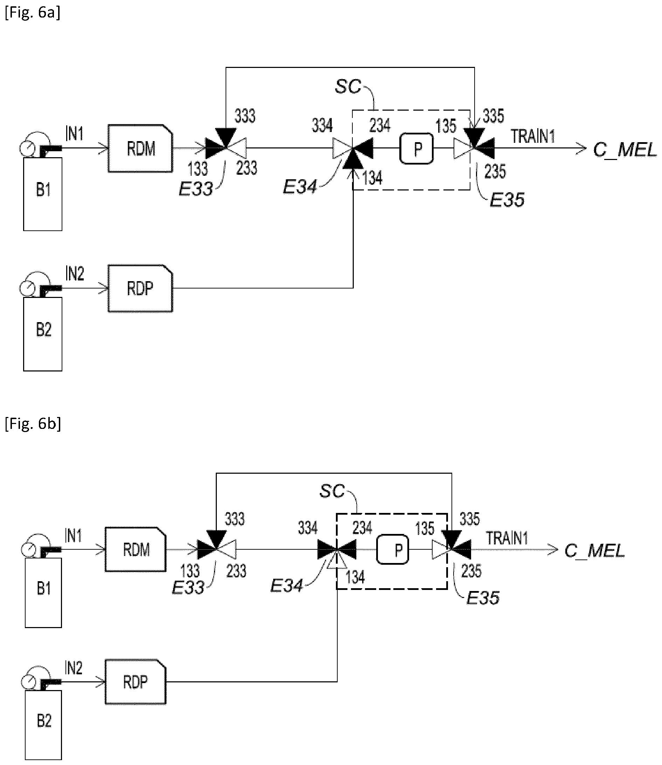

Le générateur comprend également une cellule de mélange C_MEL comprenant au moins une zone tampon Z1 (la cellule sera détaillée en [Fig. 6a] à [Fig. 6e]). La cellule de mélange C_MEL comprend une entrée IN_MEL et une sortie OUT_MEL de gaz et sa sortie forme la sortie du générateur. La seconde voie B de l'électrovanne à 6 voies E6 est reliée à une entrée de la cellule de mélange C_MEL.

Les quatrième D et cinquième E voies de l'électrovanne à 6 voies E6 sont reliées entre elles.

Lors de l'étape a), à l'intérieur de l'électrovanne à 6 voies E6, la première voie A est reliée à la troisième voie C, tandis que la sixième voie F est reliée à la cinquième voie E et la quatrième voie D est reliée à la seconde voie B. De plus, la première voie 1 de l'électrovanne à 3 voies E3 est fermée tandis que ses seconde 2 et troisième 3 voies sont ouvertes. Cela permet de faire circuler le gaz arrivant sur l'entrée IN1 du générateur dans tout le circuit microfluidique CMF du générateur. Avantageusement, le gaz envoyé en entrée IN1 est de l'air pur. Le gaz fourni à cette entrée IN1 peut venir d'une bouteille de gaz ou de l'air ambiant préalablement purifié.

Avantageusement, le générateur comprend un système informatique PC permettant de piloter le générateur et de recevoir en entrée les mesures du capteur de pression P, les valeurs de débit du régulateur de débit massique RDM et les valeurs de pression du régulateur de pression RDP, pour contrôler les deux régulateurs RDM et RDP et pour contrôler les ouvertures des voies des électrovannes (E6 et E3) du générateur. Ce système informatique PC est détaillé après la description des figures.

[Fig. 2b] et [Fig. 2c] présentent le fonctionnement du même générateur lors de l'étape b). Durant cette étape, dans un premier temps ([Fig. 2b]), la première voie A de l'électrovanne à 6 voies E6 est reliée à la seconde voie B de l'électrovanne E6. Cela permet de former et d'envoyer un premier train d'air TRAIN1, du gaz venant de la première entrée IN1 du générateur, vers la cellule de mélange C_MEL. Les quatrième D et cinquième E voies de l'électrovanne à 6 voies E6 sont reliées entre elles en circuit fermé. La troisième C voie de l'électrovanne E6 est reliée à la sixième voie F de cette même électrovanne. La seconde voie 2 de l'électrovanne à 3 voies E3 est fermée tandis que la première 1 et la troisième 3 voies de cette électrovanne E3 sont ouvertes. Ainsi, le polluant gazeux arrivant sur la seconde entrée IN2 du générateur est ajouté dans le sous-circuit SC, qui est fermé.

Puis afin d'homogénéiser la pression en gaz venant de l'entrée IN2, on ferme la voie 1 de l'électrovanne à 3 voies E3 et on ouvre la voie 2, la voie 3 restant ouverte. Cela est représenté sur la [Fig. 2c]. On envoie toujours le premier train d'air TRAIN1 vers la cellule de mélange C_MEL.

[Fig. 2d] représente la dernière étape d'un cycle (étape c)) dans laquelle on ouvre le sous-circuit SC de manière à ce qu'il envoie un second train d'air TRAIN2 vers la cellule de mélange C_MEL. Pour cela, les entrées 2 et 3 de l'électrovanne à 3 voies E3 sont toujours ouvertes et la première entrée 1 est fermée. La première voie A de l'électrovanne à 6 voies est reliée à la troisième voie C tandis que la sixième voie F est reliée à la cinquième voie E et la quatrième voie D est reliée à la seconde voie B. Du gaz est toujours envoyé vers la première entrée IN1 du générateur, ce qui a pour effet de chasser le gaz compris dans le sous-circuit SC vers la cellule de mélange C_MEL.

La cellule de mélange C_MEL reçoit alors les trains d'air TRAIN1 et TRAIN2 l'un après l'autre et a pour rôle de mélanger ces deux trains d'air afin qu'en sortie de la cellule de mélange, on obtienne un mélange gazeux homogène contenant à la fois du gaz du premier train d'air TRAIN1 et du gaz du second train d'air TRAIN2.

Le gaz envoyé sur la seconde entrée IN2 du générateur est préférentiellement un composé organique volatil (COV).

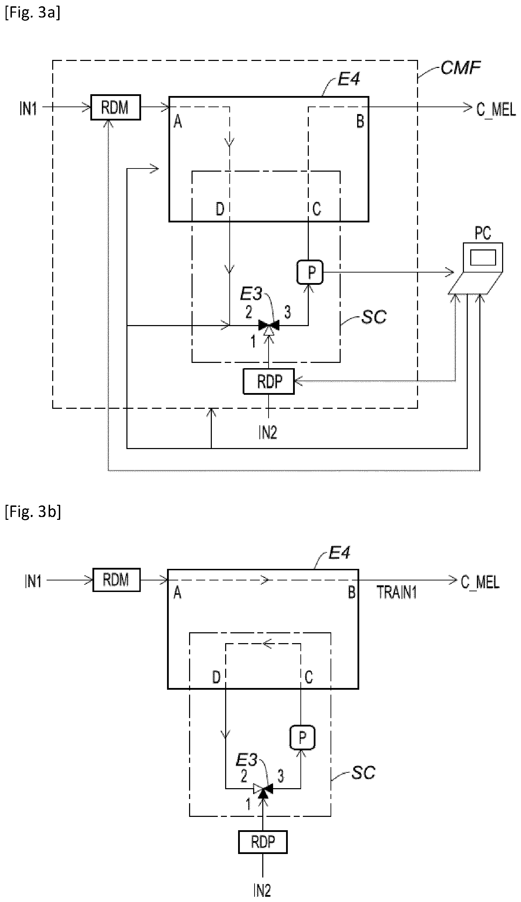

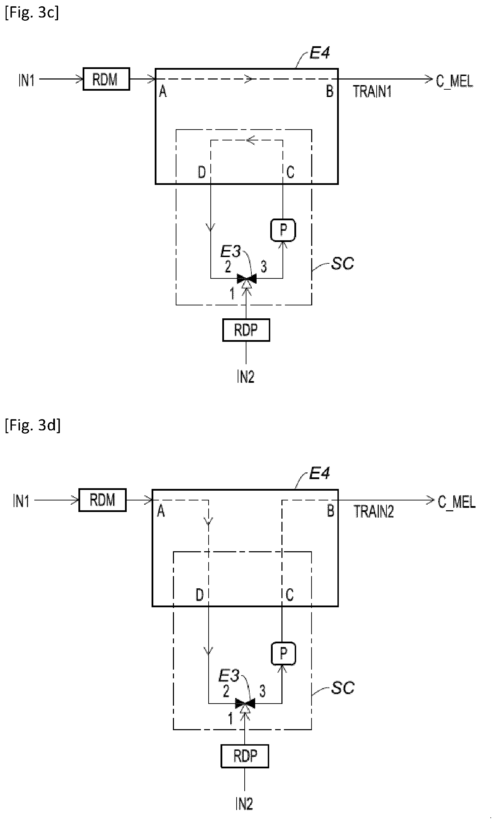

[Fig. 3a] à [Fig. 3d] présentent la mise en oeuvre du procédé selon l'invention avec un second type de générateur.

[Fig. 3a] présente le fonctionnement d'un second type de générateur lors de l'étape a), dite de purge, du procédé. Le générateur comprend un régulateur de débit massique RDM placé à une première entrée IN1 du générateur. Ce régulateur RDM est relié à une première voie A d'une électrovanne à 4 voies E4. La seconde voie B de l'électrovanne à 4 voies E4 est reliée à l'entrée d'une cellule de mélange C_MEL comprise dans le générateur. La sortie de la cellule de mélange C_MEL formant une sortie du générateur. La première voie A de l'électrovanne à 4 voies est reliée à la quatrième voie D de l'électrovanne E4 et la troisième voie C de E4 est reliée à la seconde voie B de la même électrovanne E4.

Une électrovanne à 3 voies E3 est également comprise dans le générateur. Sa première voie 1 est reliée à une seconde entrée IN2 du générateur, tandis que sa seconde voie 2 est reliée à la quatrième voie D de l'électrovanne à 4 voies E4 et sa troisième voie 3 est reliée à la troisième voie C de l'électrovanne à 4 voies E4. Un capteur de pression P est également présent dans le générateur et est placé entre la troisième voie 3 de l'électrovanne E3 et la troisième voie C de l'électrovanne E4. Il permet de mesurer la pression en gaz arrivant sur l'entrée IN2 du générateur dans le sous-circuit SC. Le sous-circuit SC est formé par le chemin parcouru par le gaz, arrivant sur la seconde entrée IN2, dans l'électrovanne à 3 voies E3 et dans les quatrième D et troisième C voies de l'électrovanne à 4 voies E4. Un régulateur de pression RDP est placé entre l'entrée IN2 et la première voie 1 de l'électrovanne E3 pour contrôler la quantité de gaz injectée dans le sous-circuit SC.

L'ensemble régulateur de débit massique RDM, régulateur de pression RDP, électrovannes à 3 et 4 voies (E3, E4) et le capteur de pression P forme le circuit microfluidique CMF du générateur.

Lors de l'étape a), à l'intérieur de l'électrovanne à 4 voies E4, la première voie A est reliée à la quatrième voie D et la troisième voie C est reliée à la seconde voie B. De plus, la première voie 1 de l'électrovanne à 3 voies E3 est fermée tandis que ses seconde 2 et troisième 3 voies sont ouvertes. Cela permet de faire circuler le gaz arrivant sur l'entrée IN1 du générateur dans tout le circuit microfluidique CMF du générateur. Avantageusement, le gaz envoyé en entrée IN1 est de l'air pur. Le gaz fourni à cette entrée IN1 peut venir d'une bouteille de gaz ou de l'air ambiant préalablement purifié.

Comme précédemment, le générateur peut comprendre un système informatique PC permettant de contrôler le générateur et de recevoir en entrée les mesures du capteur de pression P et les valeurs de débit ou de pression des régulateurs RDM et RDP. Ce système informatique PC est détaillé après la description des figures.

[Fig. 3b] et [Fig. 3c] présentent le fonctionnement du même générateur lors des deux phases de l'étape b). Durant cette étape, dans un premier temps ([Fig. 3b]), la première voie A de l'électrovanne à 4 voies E4 est reliée à la seconde voie B de l'électrovanne E4. Cela permet de former et d'envoyer un premier train d'air TRAIN1, du gaz venant de la première entrée IN1 du générateur, vers la cellule de mélange C_MEL. La troisième C voie de l'électrovanne E4 est reliée à la quatrième voie D de cette même électrovanne. La seconde voie 2 de l'électrovanne à 3 voies E3 est fermée tandis que la première 1 et la troisième 3 voies de cette électrovanne E3 sont ouvertes. Ainsi, le gaz arrivant sur la seconde entrée IN2 du générateur est ajouté dans le sous-circuit SC, qui est fermé.

Puis afin d'homogénéiser la pression en gaz venant de l'entrée IN2, on ferme la voie 1 de l'électrovanne à 3 voies E3 et on ouvre la voie 2. Cela est représenté sur la [Fig. 3c]. On envoie toujours le premier train d'air TRAIN1 vers la cellule de mélange C_MEL.

[Fig. 3d] représente la dernière étape d'un cycle (étape c)) du procédé dans laquelle on ouvre le sous-circuit SC de manière à ce qu'il envoie un second train d'air TRAIN2 vers la cellule de mélange C_MEL. Pour cela, les entrées 2 et 3 de l'électrovanne à 3 voies E3 sont toujours ouvertes et la première entrée 1 est fermée. La première voie A de l'électrovanne à 4 voies E4 est reliée à la quatrième voie D et la troisième voie C est reliée à la seconde voie B. Du gaz est toujours envoyé vers la première entrée IN1 du générateur, ce qui a pour effet d'entraîner le gaz compris dans le sous-circuit SC vers la cellule de mélange C_MEL.

La cellule de mélange C_MEL reçoit alors les trains d'air TRAIN1 et TRAIN2 l'un après l'autre et a pour rôle de mélanger ces deux trains d'air afin qu'en sortie de la cellule de mélange, on obtienne un mélange gazeux homogène contenant à la fois du gaz du premier train d'air TRAIN1 et du gaz du second train d'air TRAIN2.

Comme précédemment, le gaz envoyé sur la seconde entrée IN2 du générateur est préférentiellement un composé volatil organique. Ce gaz peut être fourni par une bouteille ou un petit réservoir de gaz sous pression branchés sur la seconde entrée IN2 du générateur.

Selon un autre mode de réalisation, dans l'étape c), pour envoyer du gaz venant de la première entrée IN1 du générateur dans le sous-circuit SC, la voie C de l'électrovanne E4 peut être reliée à la voie A et la voie D à la voie B de E4.

Par rapport à un générateur comprenant une électrovanne à 6 voies, un générateur comprenant une électrovanne à 4 voies sera moins encombrant. Néanmoins, l'utilisation d'une électrovanne à 6 voies dans le générateur permet d'envisager la génération de mélange gazeux avec deux gaz différents, ces gaz étant le polluant ou un gaz porteur comprenant un polluant.

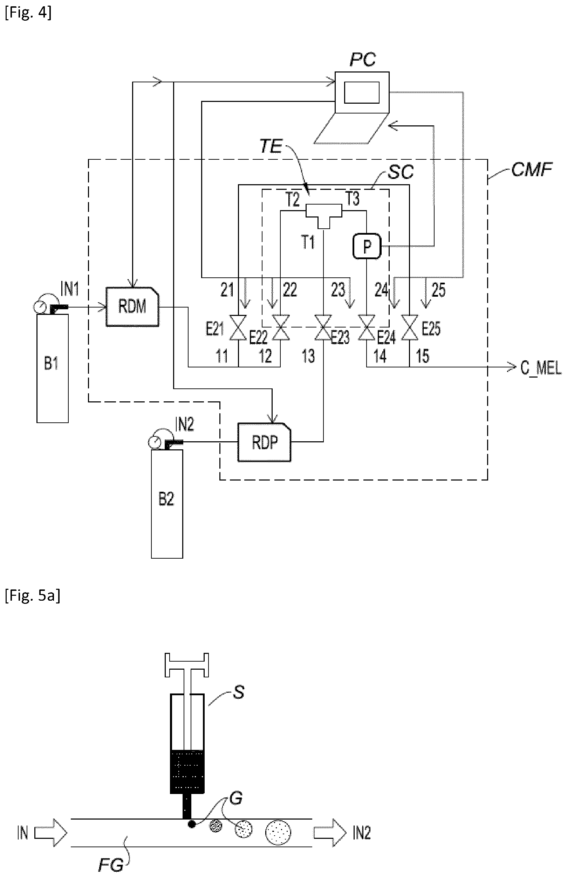

[Fig. 4] représente un troisième type de générateur de mélange gazeux pour la mise en oeuvre du procédé selon l'invention. Ce générateur comprend un régulateur de débit massique RDM relié à une première entrée IN1 du générateur. Un régulateur de pression RDP est relié à une seconde entrée IN2 du générateur. Le régulateur de pression RDP permet de réguler le flux du gaz arrivant sur la seconde entrée IN2 du générateur.

Le générateur comprend également cinq électrovannes à 2 voies (E21, E22, E23, E24, E25), un raccord en té TE, un capteur de pression et une cellule de mélange C_MEL. La sortie du régulateur massique RDM est reliée à la première voie 11 de la première électrovanne E21. La seconde voie 21 de la première électrovanne E21 est reliée à la seconde voie 25 de la cinquième électrovanne E25 et la première voie 15 de la cinquième électrovanne E25 est reliée à l'entrée de la cellule de mélange C_MEL.

Le régulateur de débit massique RDM est également relié à la première voie 12 de la seconde électrovanne E22. La première voie 14 de la quatrième électrovanne E24 est également reliée à l'entrée de la cellule de mélange C_MEL. Le régulateur de pression RDP est placé entre la seconde entrée du générateur IN2 et la première voie 13 de la troisième électrovanne E23.

La seconde voie 22 de la seconde électrovanne E22 est reliée à la seconde entrée T2 du raccord en té TE, la seconde voie 23 de la troisième électrovanne E23 est reliée à la première entrée T1 du raccord en té TE et la seconde voie 24 de la quatrième électrovanne E24 est reliée à la troisième entrée T3 du raccord en té TE. Le capteur de pression P est placé entre la seconde voie 24 de la quatrième électrovanne E24 et la troisième entrée T3 du raccord TE.

Lors de l'étape a) et de l'étape b), les voies des électrovannes suivantes sont ouvertes, les autres étant fermées :
- voies 11 et 21 de l'électrovanne E21 ;
- voies 15 et 25 de l'électrovanne E25 ;
- voies 12 et 22 de l'électrovanne E22 ;
- voies 14 et 24 de l'électrovanne E24 ; et
- voies 13 et 23 de l'électrovanne E23.

Puis pour homogénéiser la pression en polluant gazeux, on ferme juste les deux voies 13 et 23 de l'électrovanne E23.

Lors de l'étape c), les deux voies (12, 22, 14, 24) des électrovannes E22 et E24 sont ouvertes.

Des bouteilles de gaz (B1, B2) peuvent également être mises aux entrées (IN1, IN2) du générateur afin de fournir le générateur en gaz.

Le générateur peut comprendre un système informatique PC permettant de contrôler le générateur et de recevoir en entrée les mesures du capteur de pression P et les valeurs de débit et de pression des régulateurs RDM et RDP. Ce système informatique PC est détaillé après la description des figures.

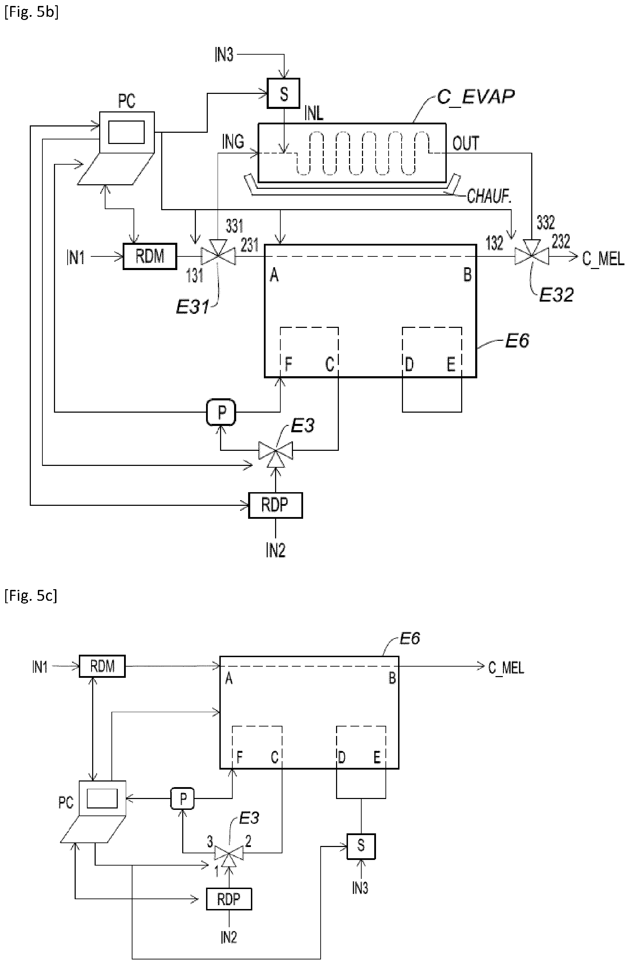

[Fig. 5a] représente une seringue S pouvant être placée en entrée de différentes voies de l'électrovanne à 6 voies E6 qui permet de déposer une ou plusieurs gouttes G d'un liquide dans un flux d'air FG. Cela permet de travailler avec des polluants liquides, comme du formaldéhyde. Pour cela, une seringue S dépose des gouttes G séquentiellement dans un flux d'air FG venant d'une entrée IN de gaz du générateur. Les gouttes G s'évaporent dans le flux d'air FG, puis le flux d'air FG comprenant les gouttes G évaporées forme le second train d'air envoyé vers la cellule de mélange. Les figures suivantes ([Fig. 5b] à [Fig. 5e]) présentent différents types de générateur permettant de générer un mélange gazeux à partir d'un polluant gazeux et/ou d'un polluant liquide.

[Fig. 5b] présente un premier type de générateur permettant générer un mélange gazeux à partir d'un polluant liquide. Le générateur comprend une électrovanne à 6 voies E6 et il comprend également deux électrovannes à 3 voies E31 et E32 et une cellule d'évaporation C_EVAP par rapport au générateur présenté dans les [Fig. 2a] à [Fig. 2d]. Il comprend également une seringue S permettant d'introduire un liquide dans le générateur par l'entrée IN3. Le régulateur de débit massique RDM est relié à la première voie 131 de l'électrovanne à 3 voies E31. La seconde voie 231 de l'électrovanne E31 est reliée à la première voie A de l'électrovanne à 6 voies E6. La troisième voie 331 de l'électrovanne E31 est reliée à une entrée en gaz ING de la cellule d'évaporation C_EVAP. La première voie 132 de l'électrovanne E32 est reliée à la seconde voie B de l'électrovanne E6, sa seconde voie 232 est reliée à l'entrée de la cellule de mélange C_MEL et sa troisième voie 332 est reliée à la sortie OUT de la cellule d'évaporation C_EVAP. La seringue S est placée de manière à pouvoir déposer des gouttes d'un polluant liquide à l'entrée d'un liquide INL de la cellule d'évaporation C_EVAP. Les gouttes déposées s'évaporent alors dans la cellule d'évaporation C_EVAP quand de l'air, issu de la première entrée IN1 du générateur, est envoyé vers la cellule C_EVAP. Ainsi le mélange comprenant le gaz issu de IN1 et les gouttes évaporées obtenu en sortie OUT de la cellule d'évaporation C_EVAP forme un second train d'air TRAIN2 (tel que celui de l'étape c) du procédé).

Lors de la formation du premier train d'air dans l'étape b), la voie 331 de l'électrovanne E31 est ouverte, de même que la voie 332 de l'électrovanne E32. Les voies 231 et 132 des électrovannes E31 et E32 sont fermées. Ce premier train d'air TRAIN1 ne comprend alors que le gaz entrant dans la première entrée IN1 du générateur. Puis, lors de l'étape c), les voies 331 et 332 restent ouvertes et les voies 231 et 132 des électrovannes E31 et E32 restent fermées, et des gouttes sont déposées grâce à la seringue S dans la cellule d'évaporation C_EVAP. Le gaz pénétrant alors dans la cellule d'évaporation C_EVAP permet d'évaporer les gouttes et ainsi d'envoyer un second train d'air vers la cellule de mélange C_MEL. Ce second train d'air TRAIN2 comprend les gouttes évaporées du polluant liquide. C'est donc l'ajout périodique des gouttes qui permet de générer alternativement les deux trains d'air l'un après l'autre, sans agir sur les positions des électrovannes E31 et E32.

La cellule d'évaporation C_EVAP peut comprendre des chicanes afin d'augmenter la distance parcourue par le mélange, constitué des gouttes venant de l'entrée INL et du gaz venant de l'entrée ING, dans la cellule d'évaporation. Plus cette distance est grande, plus les gouttes ont de temps pour s'évaporer dans le flux d'air avant de ressortir de la cellule C_EVAP.

Par ailleurs, afin d'accélérer l'évaporation des gouttes dans la cellule d'évaporation C_EVAP, le générateur peut comprendre des moyens de chauffage CHAUF. de la cellule d'évaporation C_EVAP. Ces moyens de chauffage CHAUF. peuvent par exemple être choisis parmi : une céramique chauffante, une résistance chauffante ou un four, le tout pouvant être isolé pour assurer une température constante.

L'électrovanne à 6 voies E6 peut être remplacée par une électrovanne à 4 voies, comme dans les [Fig. 3a] à [Fig. 3d].

Le générateur peut comprendre un système informatique PC permettant de contrôler le générateur, notamment les ouvertures des différentes voies des électrovannes (E3, E31, E32 et E6), les régulateurs RDP et RDM et l'actionneur de la seringue S, de recevoir en entrée les mesures du capteur de pression P, les valeurs de débit et de pression des régulateurs RDM et RDP. Ce système informatique PC est détaillé après la description des figures.

[Fig. 5c] et [Fig. 5d] présentent un autre type de générateur permettant de générer un mélange gazeux à partir d'un polluant gazeux et/ou liquide. Le générateur présenté comprend une électrovanne à 6 voies comme dans les [Fig. 2a] à [Fig. 2d], et il comprend, en plus, une seringue S reliée aux voies D et E de l'électrovanne à 6 voies E6. Un polluant liquide peut ainsi être déposé dans le générateur par une troisième entrée IN3 du générateur.

[Fig. 5c] présente ce générateur durant l'étape b) du procédé durant laquelle le liquide est déposé goutte à goutte dans le sous-circuit fermé formé par les voies D et E de l'électrovanne E6. Puis dans la [Fig. 5d], le sous-circuit est ouvert et la voie E est reliée à la voie F et la voie D est reliée à la voie B de l'électrovanne E6. Ainsi les gouttes déposées durant l'étape b) vont s'évaporer dans le flux d'air arrivant de la première entrée IN1 du générateur et un second train d'air TRAIN2 comprenant ces gouttes évaporées est envoyé vers la cellule de mélange C_MEL.

Le générateur peut comprendre un système informatique PC permettant de contrôler le générateur, notamment les ouvertures des différentes voies des électrovannes (E3 et E6) et de recevoir en entrée les mesures du capteur de pression P. Ce système informatique PC est détaillé après la description des figures.

Le générateur décrit dans ces deux figures peut également comprendre un moyen de chauffage, placé au niveau des voies D et E de l'électrovanne E6, permettant d'accélérer l'évaporation des gouttes dans le sous-circuit formé par les voies D et E.

[Fig. 5e] présente encore un autre type de générateur permettant de générer un mélange gazeux à partir d'un polluant liquide ou gazeux. Le générateur présenté comprend une électrovanne à 6 voies E6 et une seringue S reliée à la voie C de cette électrovanne E6. Dans cet exemple, l'électrovanne E6 pourrait également être une électrovanne à 4 voies. Comme précédemment, des gouttes de liquide sont déposées dans le générateur grâce à la seringue S dans un sous-circuit. Ici, le sous-circuit est formé par les voies F et C de l'électrovanne E6, puis à l'ouverture du sous-circuit, les gouttes s'évaporent dans le flux d'air venant de la première entrée IN1 du générateur pour former un second train d'air TRAIN2. Ce générateur peut également comprendre un moyen de chauffage, placé au niveau des voies F et C de l'électrovanne E6, permettant d'accélérer l'évaporation des gouttes dans le sous-circuit formé par les voies F et C.

Dans ces différents types de générateur, il est tout à fait envisageable de supprimer tous les éléments permettant de recevoir du polluant gazeux en entrée IN2 du générateur et de ne garder que les éléments permettant de recevoir un polluant liquide par l'entrée IN3 du générateur.

Plus généralement, la seringue S peut être remplacée par tout dispositif de génération de gouttes, comme par exemple une tête d'impression ou une puce microfluidique.

Selon un autre mode de réalisation de l'invention, les générateurs décrits précédemment comprennent un système de purification placé à la première entrée IN1 du générateur. Ce système de purification permet de purifier l'air notamment si le gaz envoyé vers l'entrée IN1 est de l'air ambiant.

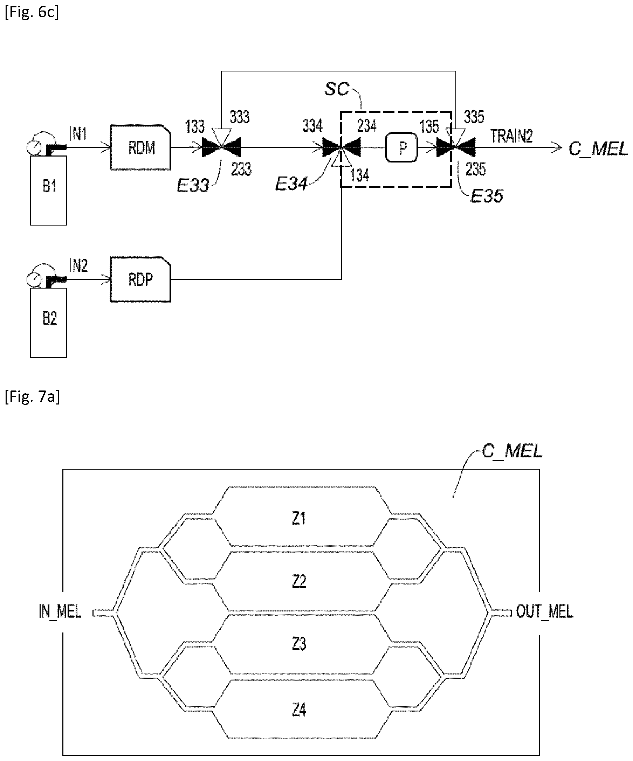

[Fig. 6a] à [Fig. 6c] présentent un quatrième type de générateur permettant de générer un mélange gazeux à partir d'un polluant gazeux. Le générateur comprend trois électrovannes à 3 voies (E33, E34, E35), un régulateur de débit massique RDM, un régulateur de pression RDP, un capteur de pression P et une cellule de mélange C_MEL. Le régulateur de débit massique est placé entre une première entrée IN1 du générateur et la première voie 133 de l'électrovanne E33. La première entrée IN1 est adaptée pour recevoir un gaz, en particulier de l'air pur. Une bouteille B1 d'air pur peut ainsi être placée à l'entrée IN1 du générateur.

Le régulateur de pression RDP est placé entre une seconde entrée IN2 du générateur et la première voie 134 de l'électrovanne E34. La seconde entrée IN2 est adaptée pour recevoir un gaz, en particulier un gaz polluant. Une bouteille B2 de polluant gazeux peut ainsi être placée en entrée IN2 du générateur.

Le capteur de pression P est placée entre la seconde voie 234 de l'électrovanne E34 et la première voie 135 de l'électrovanne E35.

La seconde voie 233 de l'électrovanne E33 est reliée à la troisième voie 334 de l'électrovanne E34. La troisième voie 333 de l'électrovanne E33 est reliée à la troisième voie 335 de l'électrovanne E35. La seconde voie 235 de l'électrovanne E35 est reliée à l'entrée de la cellule de mélange C_MEL.

[Fig. 6a] représente la première phase de l'étape b), c'est-à-dire ce qu'il se passe pendant tᵢₙⱼ. Les voies 133 et 333 de l'électrovanne E33, les voies 134 et 234 de l'électrovanne E34 et les voies 335 et 235 de l'électrovanne E35 sont ouvertes, tandis que les autres voies sont fermées. Un premier train d'air pur TRAIN1 se forme et est envoyé vers la cellule de mélange C_MEL. Du gaz polluant est ajouté dans le sous-circuit SC qui est fermé.

[Fig. 6b] représente la seconde phase de l'étape b), c'est-à-dire ce qu'il se passe pendant tₕₒₘₒ. Les voies 133 et 333 de l'électrovanne E33, les voies 334 et 234 de l'électrovanne E34 et les voies 335 et 235 de l'électrovanne E35 sont ouvertes, tandis que les autres voies sont fermées. On envoie toujours un premier train d'air pur TRAIN1 vers la cellule de mélange C_MEL. On n'ajoute plus de polluant gazeux dans le sous-circuit SC et le sous-circuit SC est ouvert, ce qui permet d'homogénéiser la pression en polluant.

[Fig. 6c] présente l'étape c) du procédé, c'est-à-dire ce qu'il se passe pendant tₚₒₗ. Les voies 133 et 233 de l'électrovanne E33, les voies 334 et 234 de l'électrovanne E34 et les voies 135 et 235 de l'électrovanne E35 sont ouvertes, tandis que les autres voies sont fermées. De l'air pur est envoyé dans le sous-circuit SC, ce qui permet de créer un second train d'air comprenant le polluant gazeux TRAIN2 qui est également envoyé vers la cellule de mélange C_MEL.

Comme pour les configurations précédentes de générateur, un système informatique PC peut être présent pour contrôler le générateur, en particulier l'ouverture des voies des électrovannes et les deux régulateurs, recevoir en entrée les mesures du capteur de pression P et les valeurs de débit ou de pression des régulateurs RDM et RDP.

[Fig. 7a] présente un premier exemple d'une cellule de mélange C_MEL d'un générateur mettant en oeuvre le procédé selon l'invention. La cellule C_MEL comprend une entrée IN_MEL et une sortie OUT_MEL. La cellule C_MEL comprend au moins une zone tampon (Z1, Z2, Z3, Z4) dans laquelle se mélangent les deux trains d'air reçus. Avantageusement, la cellule C_MEL comprend quatre zones tampon (Z1, Z2, Z3, Z4) mises en parallèle, chaque zone comprenant une entrée reliée à l'entrée IN_MEL de la cellule C_MEL et une sortie reliée à la sortie OUT_MEL de la cellule C_MEL.

Quand un train d'air pénètre dans la cellule de mélange C_MEL, il se répartit dans les quatre zones tampon, ce qui permet de favoriser le mélange homogène des deux trains d'air reçus par la cellule C_MEL.

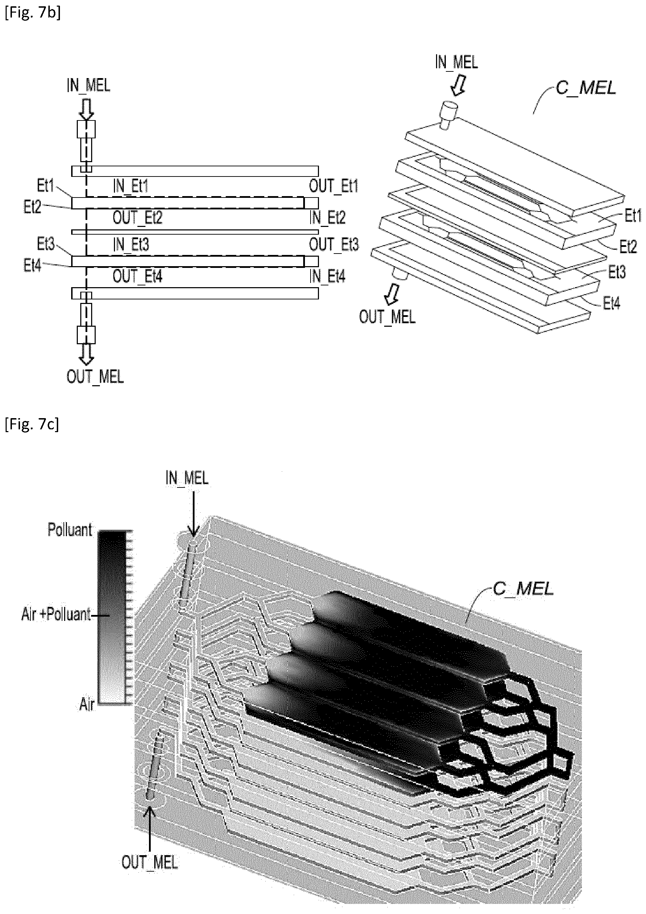

[Fig. 7b] présente une vue en coupe et en trois dimensions d'un second exemple d'une cellule de mélange C_MEL. Cette cellule comprend quatre étages (Et1, Et2, Et3 et Et4) dans lequel au moins une zone tampon est présente. Avantageusement, sur chaque étage, on compte au moins deux zones tampon mises en parallèle de la même façon que présenté en [Fig. 5a]. L'entrée IN_Et1 du premier étage Et1 est reliée à l'entrée IN_MEL de la cellule de mélange C_MEL et la sortie OUT_Et4 du quatrième étage Et4 est reliée à la sortie OUT_MEL de la cellule C_MEL. La sortie d'un étage intermédiaire est reliée à l'entrée de l'étage suivant.

La multiplication des zones tampon et/ou des étages dans la cellule de mélange permet d'améliorer l'homogénéité du mélange gazeux obtenu en sortie de la cellule de mélange C_MEL (c'est-à-dire obtenir un mélange gazeux présentant une concentration constante en polluant). Plus le débit de gaz en entrée IN1 du générateur est important, plus la cellule comprendra de zones tampon et/ou d'étages. Par ailleurs, si l'on ne souhaite pas avoir plusieurs étages dans la cellule de mélange, il est également possible d'augmenter les dimensions des zones tampon (profondeur et/ou largeur de la zone).

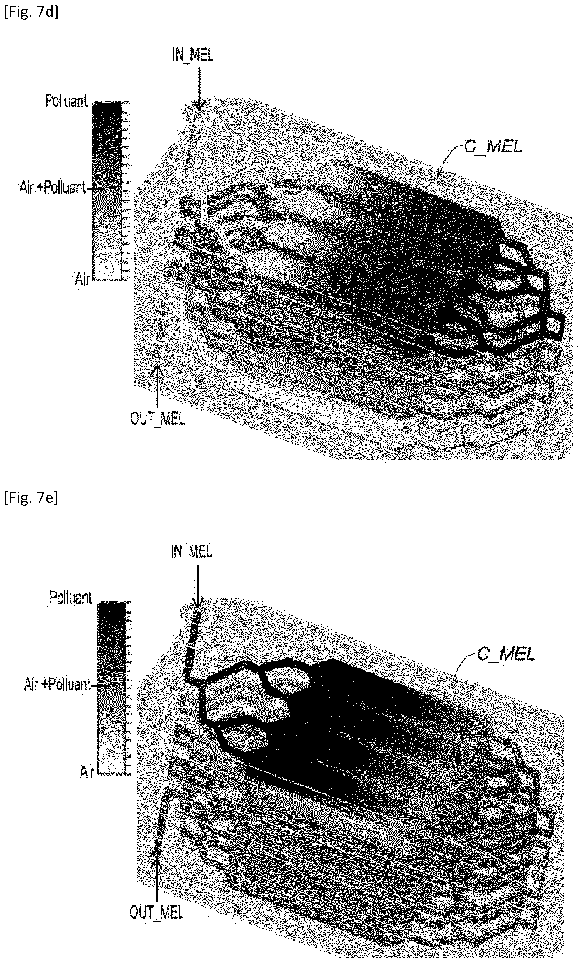

[Fig. 7c] à [Fig. 7e] présentent la cellule de mélange C_MEL au cours du temps, la [Fig. 7c] présentant la cellule à t₀, la [Fig. 7d] à t₁ et la [Fig. 7e] à t₂ avec t₀ < t₁ < t₂. Sur ces trois figures, la concentration en polluant est représentée en nuance de gris : de l'air ne comprenant que le second train d'air, ici du polluant est visible en noir sur les figures; de l'air ne comprenant que le premier train d'air TRAIN1, ici de l'air pur, est visible en blanc sur les figures ; et de l'air comprenant un mélange homogène des deux trains d'air est visible en gris sur les figures. A t₀ ([Fig. 7c]), la cellule de mélange C_MEL reçoit un second train d'air TRAIN2 comprenant du polluant, tandis que sa sortie ne comprend que de l'air pur. A t₁ ([Fig. 7d]), on peut voir que le second train d'air se propage dans la cellule et qu'il commence à se mélanger avec l'air pur déjà présent dans la cellule de mélange (les étages situées au milieu de la cellule étant devenus gris). La sortie de la cellule C_MEL ne comprend encore que de l'air pur. Un « premier train d'air » comprenant de l'air pur arrive également sur la cellule de mélange à t₁. A t₂ ([Fig. 7e]), la sortie de la cellule de mélange C_MEL est un mélange homogène des deux trains d'air reçus.

Les zones tampon et les canaux d'entrée et de sortie de la cellule de mélange peuvent être obtenus par gravure d'un substrat, par micro-fraisage, par impression en trois dimensions, ou plus généralement par toutes les techniques de réalisation de circuits microfluidiques, telles que le moulage ou la lithographie. De plus, les matériaux constituant la cellule de mélange sont des matériaux inertes ne générant pas de polluant et ne réagissant pas au contact des polluants fournis en entrée IN2 ou IN3 du générateur. Ces matériaux peuvent par exemple être choisis parmi le verre, le polyétheréthercétone (PEEK), le polyméthacrylate de méthyle (PMMA) ou le polytétrafluoroéthylène (PTFE).

Les générateurs présentés comprennent de nombreuses électrovannes dont il faut pouvoir contrôler l'ouverture et la fermeture des voies le plus précisément possible afin de ne pas perturber les cycles et les étapes du procédé. Ainsi, les générateurs peuvent comprendre un système informatique (représenté sur les figures [Fig. 2a], [Fig. 3a], [Fig. 4], [Fig. 5b] et [Fig. 5c] par la référence PC) qui pilote le générateur, et est configuré pour recevoir en entrée des mesures du capteur de pression, des valeurs de débit du régulateur de débit massique RDM et des valeurs de pression du régulateur de pression RDP, pour contrôler le régulateur de débit massique et le régulateur de pression et pour contrôler les ouvertures des voies des électrovannes du générateur. Le système informatique PC contrôle également le dispositif de génération de gouttes S.

Ainsi, le système informatique PC est capable de détecter d'éventuelles fuites de gaz dans le sous-circuit en analysant les mesures du capteur, par exemple avant la mise en fonctionnement du générateur (donc avant l'étape a)), si le capteur mesure des variations de pression ou lors de l'étape b) ou c), si la pression mesurée chute brutalement.

Ce système informatique peut également permettre de paramétrer et d'automatiser les cycles. De plus, grâce aux mesures de pression du capteur de pression, le système peut également calculer la concentration en polluant du mélange gazeux en sortie du générateur, à condition qu'on lui fournisse la concentration du liquide ajouté goutte à goutte ou la concentration en polluant fourni en entrée du générateur.

Avantageusement, le système informatique comprend trois horloges internes (hm, h1, h2 représentées en [Fig. 2a]) : une horloge maîtresse (hm) et deux horloges secondaires (h1, h2). L'horloge maîtresse (hm) a pour objectif de cadencer l'ensemble des horloges secondaires. Une des horloges secondaires (h1) permet de piloter les électrovannes, en particulier les électrovannes intervenant durant un cycle de génération de mélange gazeux, c'est-à-dire les vannes E3, E4, E6 et E21 à E25, tandis que l'autre horloge secondaire (h2) s'occupe de l'acquisition et de l'éventuel affichage des mesures du capteur de pression.

La description du procédé ainsi que des générateurs ne traite que d'un polluant, mais il est tout à fait envisageable de fournir plusieurs polluants gazeux avec des concentrations identiques ou différentes en fonction de l'échantillon gazeux ou liquide injecté en entrée IN2 ou IN3 afin de générer un mélange gazeux complexes dont on pourra calculer la concentration en sortir pour chacun des polluants.

## Revendications

1. Procédé de génération d'un mélange gazeux au moyen d'un appareil comprenant au moins deux entrées, dont la première est une entrée pour un gaz porteur (IN1) et la seconde est une entrée pour un polluant (IN2, IN3) et au moins une sortie de gaz (OUT_MEL), un système d'électrovannes (E3, E4, E6, E21, E22, E23, E24, E25, E31, E32, E33, E34, E35), un circuit microfluidique (CMF) et une cellule de mélange (C_MEL), le circuit microfluidique comprenant un sous-circuit (SC) pouvant être isolé ou mis en relation avec la cellule de mélange grâce au système d'électrovannes, **caractérisé en ce qu'**il comprend les étapes suivantes :
a) Nettoyage du circuit microfluidique par de l'air pur reçu sur la première entrée ;
b) Formation d'un premier train d'air (TRAIN1) avec un gaz reçu sur la première entrée de l'appareil, envoi de ce premier train d'air vers la cellule de mélange et ajout d'un polluant dans le sous-circuit isolé de la cellule de mélange à partir d'au moins un polluant reçu sur la seconde entrée de l'appareil ;
c) Ouverture du sous-circuit par le système d'électrovannes, de manière à ce que le sous-circuit soit relié à la première entrée de l'appareil alimentée en gaz et à l'entrée (IN_MEL) de la cellule de mélange, l'ouverture du sous-circuit provoquant l'envoi d'un second train d'air (TRAIN2) vers la cellule de mélange ;
les étapes b) et c) étant répétées jusqu'à obtenir la quantité de mélange gazeux souhaitée en sortie (OUT_MEL) de la cellule de mélange.

2. Procédé de génération d'un mélange gazeux selon la revendication 1 dans lequel le polluant ajouté dans le sous-circuit isolé durant l'étape b) est gazeux et l'étape b) comprend également l'homogénéisation de la pression en polluant gazeux dans le sous-circuit.

3. Procédé de génération d'un mélange gazeux selon la revendication 1 dans lequel le polluant ajouté durant l'étape b) est liquide et l'ajout de ce polluant est réalisé par le dépôt d'une goutte (G) ou le dépôt séquentiel d'au moins deux gouttes du polluant dans le sous-circuit et durant l'étape c), l'ouverture du sous-circuit provoque l'évaporation des gouttes dans le gaz venant de la première entrée de l'appareil.

4. Générateur de mélange gazeux pour la mise en oeuvre du procédé selon la revendication 1 comprenant :
- un régulateur de débit massique (RDM) placé à une première entrée de gaz (IN1) du générateur ;
- une première électrovanne 3 voies (E3) dont la première voie (1) est placée à une seconde entrée de gaz (IN2) du générateur ;
- un régulateur de pression (RDP) placé entre la première voie de l'électrovanne 3 voies et la seconde entrée de gaz du générateur ;
- une cellule de mélange (C_MEL) présentant une entrée (IN_MEL) et une sortie (OUT_MEL) et comprenant au moins une zone tampon (Z1, Z2, Z3, Z4) comprenant une entrée et une sortie, l'entrée de la zone tampon étant reliée à l'entrée de la cellule et la sortie de la zone tampon étant reliée à la sortie de la cellule et la sortie de la cellule formant une sortie d'un mélange gazeux du générateur ; et
- une électrovanne 6 voies (E6) dont une première voie (A) est reliée à une sortie du régulateur de débit massique, une seconde voie (B) est reliée à l'entrée de la cellule de mélange, une troisième voie (C) est reliée à une seconde voie (2) de la première électrovanne 3 voies, une quatrième (D) et une cinquième (E) voies sont reliées ensemble et une sixième voie (F) est reliée à la troisième voie (3) de l'électrovanne 3 voies.

5. Générateur de mélange gazeux selon la revendication 4 comprenant également :
- une cellule d'évaporation (C_EVAP) présentant une entrée de gaz (ING), une entrée de liquide (INL) et une sortie de gaz (OUT) ;
- une seconde (E31) et une troisième (E32) électrovannes 3 voies, une première voie (131) de la seconde électrovanne 3 voies (E31) étant reliée au régulateur de débit massique (RDM), une seconde voie (231) de la seconde électrovanne 3 voies (E31) étant reliée à la première (A) voie de l'électrovanne 6 voies, une troisième voie (331) de la seconde électrovanne 3 voies (E31) étant reliée à l'entrée de gaz (ING) de la cellule d'évaporation (C_MEL), une première voie (132) de la troisième électrovanne (E32) étant reliée à la seconde voie (B) de l'électrovanne 6 voies (E6), une seconde voie (232) de la troisième électrovanne (E32) étant reliée à l'entrée de la cellule de mélange (C_MEL) et une troisième voie (332) de la troisième électrovanne 3 voies (E32) étant reliée à la sortie (OUT) de la cellule d'évaporation (C_EVAP) ; et
- un dispositif de génération de gouttes (S) placé à l'entrée de liquide (INL) de la cellule de mélange et configurée de manière à former une entrée du générateur pour un liquide (IN3).

6. Générateur de mélange gazeux selon la revendication 4 comprenant également un dispositif de génération de gouttes (S) relié à la quatrième voie (D) de l'électrovanne 6 voies (E6) et à la cinquième voie (E) de l'électrovanne 6 voies (E6), et configurée de manière à former une entrée du générateur pour un liquide (IN3).

7. Générateur de mélange gazeux selon la revendication 4 comprenant également un dispositif de génération de gouttes (S) relié à la troisième voie (C) de l'électrovanne 6 voies (E6) et à la seconde voie (2) de la première électrovanne 3 voies (E3), et configurée de manière à former une entrée du générateur pour un liquide (IN3).

8. Générateur de mélange gazeux selon l'une des revendications 5 à 7 dans lequel le dispositif de génération de gouttes est choisi parmi une seringue, une tête d'impression ou une puce microfluidique.

9. Générateur de mélange gazeux pour la mise en oeuvre du procédé selon la revendication 1 comprenant :
- un régulateur de débit massique (RDM) placé à une première entrée (IN1) de gaz du générateur ;
- une première électrovanne 3 voies (E3) dont la première (1) voie est placée à une seconde entrée (IN2) de gaz du générateur ;
- un régulateur de pression (RDP) placé entre la première voie de l'électrovanne 3 voies et la seconde entrée de gaz du générateur ;
- une cellule de mélange (C_MEL) présentant une entrée (IN_MEL) et une sortie (OUT_MEL) et comprenant au moins une zone tampon (Z1, Z2, Z3, Z4) comprenant une entrée et une sortie, l'entrée de la zone tampon étant reliée à l'entrée de la cellule et la sortie de la zone tampon étant reliée à la sortie de la cellule et la sortie de la cellule formant une sortie d'un mélange gazeux du générateur ; et
- une électrovanne 4 voies (E4) dont une première voie (A) est reliée à une sortie du régulateur de débit massique, une seconde voie (B) est reliée à une entrée de la cellule de mélange, une troisième voie (C) est reliée à une seconde voie (2) de la première électrovanne 3 voies et une quatrième voie (D) est reliée à une troisième voie (3) de la première électrovanne 3 voies.

10. Générateur de mélange gazeux pour la mise en oeuvre du procédé selon la revendication 1 comprenant :
- un régulateur de débit massique (RDM) relié à une première entrée (IN1) du générateur ;
- une cellule de mélange (C_MEL) présentant une entrée (IN_MEL) et une sortie (OUT_MEL) et comprenant au moins une zone tampon (Z1, Z2, Z3, Z4) comprenant une entrée et une sortie, l'entrée de la zone tampon étant reliée à l'entrée de la cellule et la sortie de la zone tampon étant reliée à la sortie de la cellule et la sortie de la cellule formant une sortie d'un mélange gazeux du générateur ;
- cinq électrovannes 2 voies (E21, E22, E23, E24, E25) ;
- un capteur de pression (P) ;
- un régulateur de pression (RDP) ; et
- un raccord en té (TE),
la sortie du régulateur de débit massique étant reliée à une première voie (11, 12) de la première (E21) et de la seconde (E22) électrovanne, une seconde voie (21) de la première électrovanne étant reliée à une première voie (25) de la cinquième électrovanne (E25), une seconde voie (22) de la seconde électrovanne (E22) étant reliée à une première entrée (T2) du raccord en té, une première voie (13) de la troisième électrovanne (E23) étant reliée à une seconde entrée du générateur (IN2), une seconde voie (23) de la troisième électrovanne (E23) étant reliée à une deuxième entrée (T1) du raccord en té, une première voie (14) de la quatrième et une seconde voie (15) de la cinquième électrovanne (E25) étant reliées à l'entrée de la cellule de mélange (C_MEL), une seconde voie (24) de la quatrième électrovanne (E24) étant reliée à la troisième entrée (T3) du raccord en té, le régulateur de pression (RDP) étant placé entre la première voie (13) de la troisième électrovanne (E23) et la seconde entrée du générateur (IN2) et le capteur de pression (P) étant placé entre la seconde voie (24) de la quatrième électrovanne (E24) et la troisième entrée (T3) du raccord en té.

11. Générateur de mélange gazeux pour la mise en oeuvre du procédé selon la revendication 1 comprenant :
- un régulateur de débit massique (RDM) placé à une première entrée de gaz (IN1) du générateur ;
- une première électrovanne 3 voies (E34) dont la première voie (134) est placée à une seconde entrée de gaz (IN2) du générateur ;
- un régulateur de pression (RDP) placé entre la première voie (134) de la première électrovanne 3 voies et la seconde entrée de gaz du générateur ;
- une seconde électrovanne 3 voies (E33) dont la première voie (133) est placée à une sortie du régulateur de débit massique et la seconde voie (233) est reliée à la troisième voie (334) de la première électrovanne ;
- une troisième électrovanne 3 voies (E35) dont la troisième voie (335) est reliée à la troisième voie (333) de la seconde électrovanne, la première voie (135) est reliée à la seconde voie (234) de la première électrovanne ; et
- une cellule de mélange (C_MEL) présentant une entrée (IN_MEL) et une sortie (OUT_MEL) et comprenant au moins une zone tampon (Z1, Z2, Z3, Z4) comprenant une entrée et une sortie, l'entrée de la zone tampon étant reliée à l'entrée de la cellule, la sortie de la zone tampon étant reliée à la sortie de la cellule, la sortie de la cellule formant une sortie d'un mélange gazeux du générateur, et l'entrée de la cellule étant reliée à la seconde voie (235) de la troisième électrovanne.

12. Générateur de mélange gazeux selon l'une des revendications 4 à 11 comprenant un capteur de pression (P) configuré de manière à mesurer la pression en polluant issu de la seconde entrée (IN2) en gaz du générateur et un système informatique (PC) pour piloter le générateur, le système informatique étant configuré pour recevoir en entrée des mesures du capteur de pression, des valeurs de débit du régulateur de débit massique (RDM), des valeurs de pression du régulateur de pression (RDP), pour contrôler le régulateur de débit massique (RDM) et le régulateur de pression (RDP) et pour contrôler les ouvertures des voies des électrovannes (E21, E22, E23, E24, E25, E3, E31, E32, E33, E35, E36, E4, E6) du générateur.

13. Générateur de mélange gazeux selon la revendication 12 comprenant un dispositif de génération de goutte (S) placé à une entrée de liquide (INL) de la cellule de mélange (C_MEL) et configurée de manière à former une entrée du générateur pour un liquide (IN3), dans lequel le système informatique est également configuré pour contrôler le dispositif de génération de gouttes.

14. Générateur de mélange gazeux selon l'une des revendications 4 à 13 dans lequel la cellule de mélange comprend au moins deux zones tampon (Z1, Z2, Z3, Z4), chacune des zones tampon étant reliées à l'entrée de la cellule de mélange (IN_MEL) et à la sortie de la cellule de mélange (OUT_MEL).

15. Générateur de mélange gazeux selon l'une des revendications 4 à 14 dans lequel la cellule de mélange est multi-étages, chaque étage (Et1, Et2, Et3, Et4) de la cellule comprenant une entrée (IN_Et1, IN_Et2, IN_Et3, IN_Et4), une sortie (OUT_Et1, OUT_Et2, OUT_Et3, OUT_Et4) et au moins une zone tampon, l'entrée d'un étage étant reliée à la sortie d'un autre étage, l'entrée du premier étage de la cellule étant reliée à l'entrée de la cellule et la sortie du dernier étage de la cellule étant reliée à la sortie de la cellule (OUT_MEL).

## Patentansprüche

1. Verfahren zur Erzeugung eines Gasgemisches mittels eines Gerätes, umfassend mindestens zwei Einlässe, von denen der erste ein Einlass für ein Trägergas (IN1) und der zweite ein Einlass für einen Schadstoff (IN2, IN3) ist, und mindestens einen Gasauslass (OUT_MEL), ein System von Magnetventilen (E3, E4, E6, E21, E22, E23, E24, E25, E31, E32, E33, E34, E35), einen mikrofluidischen Kreislauf (CMF) und eine Mischzelle (C_MEL), wobei der mikrofluidische Kreislauf einen Teilkreislauf (SC) umfasst, der durch das Magnetventilsystem isoliert oder mit der Mischzelle in Verbindung gebracht werden kann, **dadurch gekennzeichnet, dass** es folgende Schritte umfasst:
a) Reinigen des mikrofluidischen Kreislaufs mit reiner Luft, die am ersten Einlass aufgenommen wird;
b) Bilden eines ersten Luftstroms (TRAIN1) mit einem Gas, das am ersten Einlass des Gerätes aufgenommen wird, Senden dieses ersten Luftstroms zur Mischzelle und Hinzufügen eines Schadstoffs in den isolierten Teilkreislauf der Mischzelle aus mindestens einem Schadstoff, der am zweiten Einlass des Gerätes aufgenommen wird;
c) Öffnen des Teilkreislaufs durch das Magnetventilsystem, so dass der Teilkreislauf mit dem ersten mit Gas versorgten Einlass des Gerätes und mit dem Einlass (IN_MEL) der Mischzelle verbunden ist, wobei das Öffnen des Teilkreislaufs bewirkt, dass ein zweiter Luftstrom (TRAIN2) zur Mischzelle gesendet wird;
wobei die Schritte b) und c) wiederholt werden, bis die gewünschte Menge an Gasgemisch am Auslass (OUT_MEL) der Mischzelle erzielt wird.

2. Verfahren zur Erzeugung eines Gasgemisches nach Anspruch 1, wobei der Schadstoff, der während Schritt b) in den isolierten Teilkreislauf hinzugefügt wird, gasförmig ist und Schritt b) ebenfalls die Homogenisierung des Drucks an gasförmigem Schadstoff in dem Teilkreislauf umfasst.

3. Verfahren zur Erzeugung eines Gasgemisches nach Anspruch 1, wobei der während Schritt b) hinzugefügte Schadstoff flüssig ist und die Hinzufügung dieses Schadstoffs durch die Ablagerung eines Tropfens (G) oder die sequenzielle Ablagerung von mindestens zwei Tropfen des Schadstoffs in dem Teilkreislauf erfolgt und während Schritt c) das Öffnen des Teilkreislaufs die Verdampfung der Tropfen in das vom ersten Einlass des Gerätes kommende Gas bewirkt.

4. Gasgemischgenerator zur Umsetzung des Verfahrens nach Anspruch 1, Folgendes umfassend:
- einen Massendurchflussregler (RDM), der an einem ersten Gaseinlass (IN1) des Generators platziert ist;
- ein erstes 3-Wege-Magnetventil (E3), dessen erster Kanal (1) an einem zweiten Gaseinlass (IN2) des Generators platziert ist;
- einen Druckregler (RDP), der zwischen dem ersten Kanal des 3-Wege-Magnetventils und dem zweiten Generatoreinlass platziert ist;
- eine Mischzelle (C MEL), die einen Einlass (IN_MEL) und einen Auslass (OUT_ MEL) aufweist und mindestens eine Pufferzone (Z1, Z2, Z3, Z4) umfasst, die einen Einlass und einen Auslass umfasst, wobei der Einlass der Pufferzone mit dem Einlass der Zelle verbunden ist und der Auslass der Pufferzone mit dem Auslass der Zelle verbunden ist und der Auslass der Zelle einen Auslass für ein Gasgemisch aus dem Generator bildet; und
- ein 6-Wege-Magnetventil (E6), von dem ein erster Kanal (A) mit einem Auslass des Massendurchflussreglers verbunden ist, ein zweiter Kanal (B) mit dem Einlass der Mischzelle verbunden ist, ein dritter Kanal (C) mit einem zweiten Kanal (2) des ersten 3-Wege-Magnetventils verbunden ist, ein vierter (D) und ein fünfter (E) Kanal miteinander verbunden sind und ein sechster Kanal (F) mit dem dritten Kanal (3) des 3-Wege-Magnetventils verbunden ist.

5. Gasgemischgenerator nach Anspruch 4, ebenfalls Folgendes umfassend:
- eine Verdampfungszelle (C_EVAP), die einen Gaseinlass (ING), einen Flüssigkeitseinlass (INL) und einen Gasauslass (OUT) aufweist;
- ein zweites (E31) und ein drittes (E32) 3-Wege-Magnetventil, wobei ein erster Kanal (131) des zweiten 3-Wege-Magnetventils (E31) mit dem Massendurchflussregler (RDM) verbunden ist, ein zweiter Kanal (231) des zweiten 3-Wege-Magnetventils (E31) mit dem ersten (A) Kanal des 6-Wege-Magnetventils verbunden ist, ein dritter Kanal (331) des zweiten 3-Wege-Magnetventils (E31) mit dem Gaseinlass (ING) der Verdampfungszelle (C_MEL) verbunden ist, ein erster Kanal (132) des dritten Magnetventils (E32) mit dem zweiten Kanal (B) des 6-Wege-Magnetventils (E6) verbunden ist, ein zweiter Kanal (232) des dritten Magnetventils (E32) mit dem Einlass der Mischzelle (C_MEL) verbunden ist und ein dritter Kanal (332) des dritten 3-Wege-Magnetventils (E32) mit dem Auslass (OUT) der Verdampfungszelle (C_EVAP) verbunden ist; und
- eine Tropfenerzeugungsvorrichtung (S), die am Flüssigkeitseinlass (INL) der Mischzelle platziert und so konfiguriert ist, dass sie einen Generatoreinlass für eine Flüssigkeit (IN3) bildet.

6. Gasgemischgenerator nach Anspruch 4, ebenfalls umfassend eine Tropfenerzeugungsvorrichtung (S), die mit dem vierten Kanal (D) des 6-Wege-Magnetventils (E6) und dem fünften Kanal (E) des 6-Wege-Magnetventils (E6) verbunden und so konfiguriert ist, dass sie einen Generatoreinlass für eine Flüssigkeit (IN3) bildet.

7. Gasgemischgenerator nach Anspruch 4, ebenfalls umfassend eine Tropfenerzeugungsvorrichtung (S), die mit dem dritten Kanal (C) des 6-Wege-Magnetventils (E6) und dem zweiten Kanal (2) des ersten 3-Wege-Magnetventils (E3) verbunden und so konfiguriert ist, dass sie einen Generatoreinlass für eine Flüssigkeit (IN3) bildet.

8. Gasgemischgenerator nach einem der Ansprüche 5 bis 7, wobei die Tropfenerzeugungsvorrichtung aus einer Spritze, einem Druckkopf oder einem mikrofluidischen Chip ausgewählt ist.

9. Gasgemischgenerator zur Umsetzung des Verfahrens nach Anspruch 1, Folgendes umfassend:
- einen Massendurchflussregler (RDM), der an einem ersten Gaseinlass (IN1) des Generators platziert ist;
- ein erstes 3-Wege-Magnetventil (E3), dessen erster (1) Kanal an einem zweiten Gaseinlass (IN2) des Generators platziert ist;
- einen Druckregler (RDP), der zwischen dem ersten Kanal des 3-Wege-Magnetventils und dem zweiten Generatoreinlass platziert ist;
- eine Mischzelle (C_MEL), die einen Einlass (IN_MEL) und einen Auslass (OUT_ MEL) aufweist und mindestens eine Pufferzone (Z1, Z2, Z3, Z4) umfasst, die einen Einlass und einen Auslass umfasst, wobei der Einlass der Pufferzone mit dem Einlass der Zelle verbunden ist und der Auslass der Pufferzone mit dem Auslass der Zelle verbunden ist und der Auslass der Zelle einen Auslass für ein Gasgemisch aus dem Generator bildet; und
- ein 4-Wege-Magnetventil (E4), von dem ein erster Kanal (A) mit einem Auslass des Massendurchflussreglers verbunden ist, ein zweiter Kanal (B) mit einem Einlass der Mischzelle verbunden ist, ein dritter Kanal (C) mit einem zweiten Kanal (2) des ersten 3-Wege-Magnetventils verbunden ist und ein vierter Kanal (D) mit einem dritten Kanal (3) des ersten 3-Wege-Magnetventils verbunden ist.

10. Gasgemischgenerator zur Umsetzung des Verfahrens nach Anspruch 1, Folgendes umfassend:
- einen Massendurchflussregler (RDM), der mit einem ersten Einlass (IN1) des Generators verbunden ist;
- eine Mischzelle (C_MEL), die einen Einlass (IN_MEL) und einen Auslass (OUT_ MEL) aufweist und mindestens eine Pufferzone (Z1, Z2, Z3, Z4) umfasst, die einen Einlass und einen Auslass umfasst, wobei der Einlass der Pufferzone mit dem Einlass der Zelle verbunden ist und der Auslass der Pufferzone mit dem Auslass der Zelle verbunden ist und der Auslass der Zelle einen Auslass für ein Gasgemisch aus dem Generator bildet;
- fünf 2-Wege-Magnetventile (E21, E22, E23, E24, E25);
- einen Drucksensor (P);
- einen Druckregler (RDP), und
- eine T-Stück-Verbindung (TE),
wobei der Auslass des Massendurchflussreglers mit einem ersten Kanal (11, 12) des ersten (E21) und des zweiten (E22) Magnetventils verbunden ist, wobei ein zweiter Kanal (21) des ersten Magnetventils mit einem ersten Kanal (25) des fünften Magnetventils (E25) verbunden ist, ein zweiter Kanal (22) des zweiten Magnetventils (E22) mit einem ersten Einlass (T2) der T-Stück-Verbindung verbunden ist, ein erster Kanal (13) des dritten Magnetventils (E23) mit einem zweiten Einlass des Generators (IN2) verbunden ist, ein zweiter Kanal (23) des dritten Magnetventils (E23) mit einem zweiten Einlass (T1) der T-Stück-Verbindung verbunden ist, ein erster Kanal (14) des vierten und ein zweiter Kanal (15) des fünften Magnetventils (E25) mit dem Einlass der Mischzelle (C_MEL) verbunden sind, wobei ein zweiter Kanal (24) des vierten Magnetventils (E24) mit dem dritten Einlass (T3) der T-Stück-Verbindung verbunden ist, wobei der Druckregler (RDP) zwischen dem ersten Kanal (13) des dritten Magnetventils (E23) und dem zweiten Einlass des Generators (IN2) platziert ist und der Drucksensor (P) zwischen dem zweiten Kanal (24) des vierten Magnetventils (E24) und dem dritten Einlass (T3) der T-Stück-Verbindung platziert ist.

11. Gasgemischgenerator zur Umsetzung des Verfahrens nach Anspruch 1, Folgendes umfassend:
- einen Massendurchflussregler (RDM), der an einem ersten Gaseinlass (IN1) des Generators platziert ist;
- ein erstes 3-Wege-Magnetventil (E34), dessen erster Kanal (134) an einem zweiten Gaseinlass (IN2) des Generators platziert ist;
- einen Druckregler (RDP), der zwischen dem ersten Kanal (134) des ersten 3-Wege-Magnetventils und dem zweiten Gaseinlass des Generators platziert ist;
- ein zweites 3-Wege-Magnetventil (E33), dessen erster Kanal (133) an einem Auslass des Massendurchflussreglers platziert ist und dessen zweiter Kanal (233) mit dem dritten Kanal (334) des ersten Magnetventils verbunden ist;
- ein drittes 3-Wege-Magnetventil (E35), dessen dritter Kanal (335) mit dem dritten Kanal (333) des zweiten Magnetventils verbunden ist, wobei der erste Kanal (135) mit dem zweiten Kanal (234) des ersten Magnetventils verbunden ist; und
- eine Mischzelle (C_MEL), die einen Einlass (IN_MEL) und einen Auslass (OUT_ MEL) aufweist und mindestens eine Pufferzone (Z1, Z2, Z3, Z4) umfasst, die einen Einlass und einen Auslass umfasst, wobei der Einlass der Pufferzone mit dem Einlass der Zelle verbunden ist, der Auslass der Pufferzone mit dem Auslass der Zelle verbunden ist, der Auslass der Zelle einen Auslass für ein Gasgemisch des Generators bildet und der Einlass der Zelle mit dem zweiten Kanal (235) des dritten Magnetventils verbunden ist.

12. Gasgemischgenerator nach einem der Ansprüche 4 bis 11, umfassend einen Drucksensor (P), der so konfiguriert ist, dass er den Druck des Schadstoffs aus dem zweiten Gaseinlass (IN2) des Generators misst, und ein Computersystem (PC) zum Steuern des Generators, wobei das Computersystem konfiguriert ist zum Empfangen, am Eingang, von Messwerten des Drucksensors, von Durchflusswerten des Massendurchflussreglers (RDM), von Druckwerten des Druckreglers (RDP), zum Steuern des Massendurchflussreglers (RDM) und des Druckreglers (RDP) und zum Steuern der Öffnungen der Kanäle der Magnetventile (E21, E22, E23, E24, E25, E3, E31, E32, E33, E35, E36, E4, E6) des Generators.

13. Gasgemischgenerator nach Anspruch 12, umfassend eine Tropfenerzeugungsvorrichtung (S), die an einem Flüssigkeitseinlass (INL) der Mischzelle (C_MEL) platziert und so konfiguriert ist, dass sie einen Einlass des Generators für eine Flüssigkeit (IN3) bildet, wobei das Computersystem ebenfalls konfiguriert ist, um die Tropfenerzeugungsvorrichtung zu steuern.

14. Gasgemischgenerator nach einem der Ansprüche 4 bis 13, wobei die Mischzelle mindestens zwei Pufferzonen (Z1, Z2, Z3, Z4) umfasst, wobei jede der Pufferzonen mit dem Einlass der Mischzelle (IN_MEL) und dem Auslass der Mischzelle (OUT_MEL) verbunden ist.

15. Gasgemischgenerator nach einem der Ansprüche 4 bis 14, wobei die Mischzelle mehrstufig ist, wobei jede Stufe (Et1, Et2, Et3, Et4) der Zelle einen Einlass (IN_Et1, IN_Et2, IN_Et3, IN_Et4), einen Auslass (OUT_Et1, OUT_Et2, OUT_Et3, OUT_Et4) und mindestens eine Pufferzone umfasst, wobei der Einlass einer Stufe mit dem Auslass einer anderen Stufe verbunden ist, der Einlass der ersten Stufe der Zelle mit dem Einlass der Zelle verbunden ist und der Auslass der letzten Stufe der Zelle mit dem Auslass der Zelle (OUT_MEL) verbunden ist.

## Claims

1. A method for generating a gaseous mixture by means of an apparatus comprising at least two inputs, of which the first is an input for a carrier gas (IN1) and the second is an input for a pollutant (IN2, IN3) and at least one gas output (OUT MEL), a system of solenoid valves (E3, E4, E6, E21, E22, E23, E24, E25, E31, E32, E33, E34, E35), a microfluidic circuit (CMF) and a mixing cell (C MEL), the microfluidic circuit comprising a sub-circuit (SC) that can be isolated or connected with the mixing cell by means of the system of solenoid valves, **characterised in that** it comprises the following steps of:
a) cleaning the microfluidic circuit by pure air received on the first input;
b) forming a first air stream (TRAIN1) with a gas received on the first input of the apparatus, sending this first air stream to the mixing cell and adding a pollutant in the sub-circuit isolated from the mixing cell from at least one pollutant received on the second input of the apparatus;
c) opening the sub-circuit by the system of solenoid valves, so that the sub-circuit is linked to the first input of the apparatus supplied with gas and to the input (IN_MEL) of the mixing cell, the opening of the sub-circuit causing a second air stream (TRAIN2) to be sent to the mixing cell;
steps b) and c) being repeated until the desired quantity of gaseous mixture is obtained at the output (OUT_MEL) of the mixing cell.

2. The method for generating a gaseous mixture according to claim 1, wherein the pollutant added in the isolated sub-circuit during the step b) is gaseous and the step b) also comprises the homogenisation of the gaseous pollutant pressure in the sub-circuit.

3. The method for generating a gaseous mixture according to claim 1, wherein the pollutant added during the step b) is liquid and the addition of this pollutant is performed by the deposition of a drop (G) or the sequential deposition of at least two drops of the pollutant in the sub-circuit and, during the step c), the opening of the sub-circuit causes the evaporation of the drops in the gas coming from the first input of the apparatus.

4. A gaseous mixture generator for the implementation of the method according to claim 1, comprising:
- a mass flow rate regulator (RDM) placed at a first gas input (IN1) of the generator;
- a first 3-way solenoid valve (E3) of which the first way (1) is placed at a second gas input (IN2) of the generator;
- a pressure regulator (RDP) placed between the first way of the 3-way solenoid valve and the second gas input of the generator;
- a mixing cell (C_MEL) having an input (IN_MEL) and an output (OUT_MEL) and comprising at least one buffer zone (Z1, Z2, Z3, Z4) comprising an input and an output, the input of the buffer zone being linked to the input of the cell and the output of the buffer zone being linked to the output of the cell and the output of the cell forming an output of a gaseous mixture from the generator; and
- a 6-way solenoid valve (E6) of which a first way (A) is linked to an output of the mass flow rate regulator, a second way (B) is linked to the input of the mixing cell, a third way (C) is linked to a second way (2) of the first 3-way solenoid valve, a fourth (D) and a fifth (E) ways are linked together and a sixth way (F) is linked to the third way (3) of the 3-way solenoid valve.

5. The gaseous mixture generator according to claim 4, also comprising:
- an evaporation cell (C_EVAP) having a gas input (ING), a liquid input (INL) and a gas output (OUT);
- a second (E31) and a third (E32) 3-way solenoid valves, a first way (131) of the second 3-way solenoid valve (E31) being linked to the mass flow rate regulator (RDM), a second way (231) of the second 3-way solenoid valve (E31) being linked to the first (A) way of the 6-way solenoid valve, a third way (331) of the second 3-way solenoid valve (E31) being linked to the gas input (ING) of the evaporation cell (C_MEL), a first way (132) of the third solenoid valve (E32) being linked to the second way (B) of the 6-way solenoid valve (E6), a second way (232) of the third solenoid valve (E32) being linked to the input of the mixing cell (C_MEL) and a third way (332) of the third 3-way solenoid valve (E32) being linked to the output (OUT) of the evaporation cell (C_EVAP); and
- a drop generation device (S) placed at the liquid input (INL) of the mixing cell and configured so as to form an input of the generator for a liquid (IN3).

6. The gaseous mixture generator according to claim 4, also comprising a drop generation device (S) linked to the fourth way (D) of the 6-way solenoid valve (E6) and to the fifth way (E) of the 6-way solenoid valve (E6), and configured so as to form an input of the generator for a liquid (IN3).

7. The gaseous mixture generator according to claim 4, also comprising a drop generation device (S) linked to the third way (C) of the 6-way solenoid valve (E6) and to the second way (2) of the first 3-way solenoid valve (E3), and configured so as to form an input of the generator for a liquid (IN3).

8. The gaseous mixture generator according to one of claims 5 to 7, wherein the drop generation device is chosen from among a syringe, a print head or a microfluidic chip.

9. A gaseous mixture generator for the implementation of the method according to claim 1, comprising:
- a mass flow rate regulator (RDM) placed at a first gas input (IN1) of the generator;
- a first 3-way solenoid valve (E3) of which the first way (1) is placed at a second gas input (IN2) of the generator;
- a pressure regulator (RDP) placed between the first way of the 3-way solenoid valve and the second gas input of the generator;
- a mixing cell (C_MEL) having an input (IN_MEL) and an output (OUT_MEL) and comprising at least one buffer zone (Z1, Z2, Z3, Z4) comprising an input and an output, the input of the buffer zone being linked to the input of the cell and the output of the buffer zone being linked to the output of the cell and the output of the cell forming an output of a gaseous mixture from the generator; and
- a 4-way solenoid valve (E4) of which a first way (A) is linked to an output of the mass flow rate regulator, a second way (B) is linked to an input of the mixing cell, a third way (C) is linked to a second way (2) of the first 3-way solenoid valve and a fourth way (D) is linked to a third way (3) of the first 3-way solenoid valve.

10. A gaseous mixture generator for the implementation of the method according to claim 1, comprising:
- a mass flow rate regulator (RDM) linked to a first input (IN1) of the generator;
- a mixing cell (C_MEL) having an input (IN_MEL) and an output (OUT_MEL) and comprising at least one buffer zone (Z1, Z2, Z3, Z4) comprising an input and an output, the input of the buffer zone being linked to the input of the cell and the output of the buffer zone being linked to the output of the cell and the output of the cell forming an output of a gaseous mixture from the generator;
- five 2-way solenoid valves (E21, E22, E23, E24, E25);
- a pressure sensor (P);
- a pressure regulator (RDP); and
- a T coupling (TE),
the output of the mass flow rate regulator being linked to a first way (11, 12) of the first (E21) and of the second (E22) solenoid valve, a second way (21) of the first solenoid valve being linked to a first way (25) of the fifth solenoid valve (E25), a second way (22) of the second solenoid valve (E22) being linked to a first input (T2) of the T coupling, a first way (13) of the third solenoid valve (E23) being linked to a second input of the generator (IN2), a second way (23) of the third solenoid valve (E23) being linked to a second input (T1) of the T coupling, a first way (14) of the fourth solenoid valve and a second way (15) of the fifth solenoid valve (E25) being linked to the input of the mixing cell (C_MEL), a second way (24) of the fourth solenoid valve (E24) being linked to the third input (T3) of the T coupling, the pressure regulator (RDP) being placed between the first way (13) of the third solenoid valve (E23) and the second input of the generator (IN2) and the pressure sensor (P) being placed between the second way (24) of the fourth solenoid valve (E24) and the third input (T3) of the T coupling.

11. A gaseous mixture generator for the implementation of the method according to claim 1, comprising:
- a mass flow rate regulator (RDM) placed at a first gas input (IN1) of the generator;
- a first 3-way solenoid valve (E34) of which the first way (134) is placed at a second gas input (IN2) of the generator;
- a pressure regulator (RDP) placed between the first way (134) of the first 3 way solenoid valve and the second gas input of the generator;
- a second 3-way solenoid valve (E33) of which the first way (133) is placed at an output of the mass flow rate regulator and the second way (233) is linked to the third way (334) of the first solenoid valve;
- a third 3-way solenoid valve (E35) of which the third way (335) is linked to the third way (333) of the second solenoid valve and the first way (135) is linked to the second way (234) of the first solenoid valve; and
- a mixing cell (C_MEL) having an input (IN_MEL) and an output (OUT_MEL) and comprising at least one buffer zone (Z1, Z2, Z3, Z4) comprising an input and an output, the input of the buffer zone being linked to the input of the cell, the output of the buffer zone being linked to the output of the cell, the output of the cell forming an output of a gaseous mixture from the generator, and the input of the cell being linked to the second way (235) of the third solenoid valve.

12. The gaseous mixture generator according to one of claims 4 to 11, comprising a pressure sensor (P) configured so as to measure the pressure of pollutant from the second gas input (IN2) of the generator and a computing system (PC) for driving the generator, the computing system being configured to receive as input measurements from the pressure sensor, flow rate values from the mass flow rate regulator (RDM), pressure values from the pressure regulator (RDP), to control the mass flow rate regulator (RDM) and the pressure regulator (RDP) and to control the openings of the ways of the solenoid valves (E21, E22, E23, E24, E25, E3, E31, E32, E33, E35, E36, E4, E6) of the generator.

13. The gaseous mixture generator according to claim 12, comprising a drop generation device (S) placed at a liquid input (INL) of the mixing cell (C_MEL) and configured so as to form an input of the generator for a liquid (IN3), wherein the computing system is also configured to control the drop generation device.

14. The gaseous mixture generator according to one of claims 4 to 13, wherein the mixing cell comprises at least two buffer zones (Z1, Z2, Z3, Z4), each of the buffer zones being linked to the input of the mixing cell (IN_MEL) and to the output of the mixing cell (OUT_MEL).

15. The gaseous mixture generator according to one of claims 4 to 14, wherein the mixing cell is multi-staged, each stage (Et1, Et2, Et3, Et4) of the cell comprising an input (IN_Et1, IN_Et2, IN_Et3, IN_Et4), an output (OUT_Et1, OUT_Et2, OUT_Et3, OUT_Et4) and at least one buffer zone, the input of one stage being linked to the output of another stage, the input of the first stage of the cell being linked to the input of the cell and the output of the last stage of the cell being linked to the output of the cell (OUT_MEL).
